# EUROPEAN PATENT APPLICATION

(11) **EP 3 777 900 A1**
(43) Date of publication of application: **17.02.2021**
(21) Application number: 19775129.0
(22) Date of filing: 15.03.2019
(51) Int. Cl.: A61L 9/00, F24F 11/65, F24F 110/60

(54) **SPATIAL ENVIRONMENT MANAGEMENT SYSTEM**

(30) Priority: 30.03.2018 JP 2018070258
(71) Applicant: DAIKIN INDUSTRIES, LTD., Osaka 530-8323 (JP)
(72) Inventor: KITAGAWA, Keita, Osaka-shi, Osaka 530-8323 (JP); HANDA, Youichi, Osaka-shi, Osaka 530-8323 (JP)
(74) Representative: Global IP Europe Patentanwaltskanzlei
(86) International application number: PCT/JP2019/010815
(87) International publication number: WO 2019/188408

(57) **Abstract**

Provided is a spatial environment management system capable of maintaining the comfortability of a spatial environment by appropriately treating an odor without depending on an odor sensor. A spatial environment management system (1) includes an acquisition unit (610), a determination unit (620), an air conditioning apparatus, and a control unit (640). The acquisition unit acquires an image of a target space and a sound of the target space. The determination unit determines whether an odor emitting object is present in the target space, based on at least one of the image and the sound acquired by the acquisition unit. The air conditioning apparatus includes an airflow producing mechanism. The control unit controls the air conditioning apparatus such that the air conditioning apparatus treats an odor when the determination unit determines that an odor emitting object is present in the target space.

## Description

### TECHNICAL FIELD

The present disclosure relates to a spatial environment management system, and more specifically to a spatial environment management system that includes a device including at least one of an airflow producing mechanism and a deodorizing mechanism for treating an odor.

### BACKGROUND ART

Patent Literature 1 (JP 10-170422 A) discloses a known odor sensor configured to detect an odor. The use of such an odor sensor allows an air conditioning apparatus and a deodorizing apparatus to treat an odor.

### SUMMARY OF THE INVENTION

### <Technical Problem>

However, such an odor sensor is incapable of detecting an odor as long as an odor substance does not reach a place where the odor sensor is installed. Consequently, when an odor emitting object is distant from the odor sensor although the odor emitting object and the odor sensor are present in the same space, the odor sensor may require a long time for detecting the odor or fail to detect the odor. In such a case, even when a device such as a deodorizing apparatus is present in the same space, the device does not operate appropriately. Consequently, the comfortability of a spatial environment is possibly lowered with regard to an odor.

Hence, there is a demand for a spatial environment management system capable of maintaining the comfortability of a spatial environment by appropriately treating an odor without depending on an odor sensor.

### <Solutions to Problem>

A first aspect provides a spatial environment management system including an acquisition unit, a determination unit, a device, and a control unit. The acquisition unit is configured to acquire at least one of an image of a target space and a sound of the target space. The determination unit is configured to determine whether an odor emitting object is present in the target space, based on at least one of the image and the sound acquired by the acquisition unit. The device includes at least one of an airflow producing mechanism and a deodorizing mechanism. The control unit is configured to control the device such that the device treats an odor when the determination unit determines that the odor emitting object is present in the target space.

According to this configuration, since the determination unit determines presence of an odor emitting object from an image and a sound, the control unit promptly controls the device including the airflow producing mechanism and the deodorizing mechanism (hereinafter, simply referred to as an "device" in some cases) even when an odor sensor is distant from the odor emitting object. In addition, even when there is no odor sensor in the target space, the control unit appropriately operates the device on the odor.

A second aspect provides the spatial environment management system according to the first aspect, wherein the determination unit is further configured to determine a type of the odor emitting object based on at least one of the image and the sound acquired by the acquisition unit. When the determination unit determines that the odor emitting object is present in the target space, the control unit is configured to control the device in accordance with a content set based on the type of the odor emitting object determined by the determination unit.

According to this configuration, the control unit appropriately operates the device in consideration of the type of the odor emitting object.

A third aspect provides the spatial environment management system according to the second aspect, wherein the control unit has a plurality of control patterns for the device. The spatial environment management system further includes a storage unit configured to store types of odor emitting objects and the control patterns corresponding to the types of odor emitting objects with the types and the control patterns being associated with each other. The control unit is configured to control the device in accordance with the control pattern corresponding to the type of the odor emitting object, determined by the determination unit, based on the information stored in the storage unit.

According to this configuration, the storage unit stores the types of the odor emitting objects and the control patterns for the device with the types of the odor emitting objects and the control patterns associated with each other. The control unit therefore promptly and appropriately operates the device in accordance with the type of the odor emitting object.

A fourth aspect provides the spatial environment management system according to any of the first to third aspects, further including an identification unit. The identification unit is configured to identify, when the determination unit determines that the odor emitting object is present in the target space, a position of the odor emitting object, based on at least one of the image and the sound acquired by the acquisition unit. The control unit is configured to control the device in accordance with a content set based on the position of the odor emitting object identified by the identification unit.

According to this configuration, since the control unit controls the device based on the position of the odor emitting object, the control unit appropriately operates the device on the odor.

A fifth aspect provides the spatial environment management system according to the fourth aspect, further including a position acquisition unit. The position acquisition unit is configured to acquire information on a position of an odor reducer configured to reduce an odor. The device includes at least the airflow producing mechanism. The control unit is configured to control the device such that air passes through or by the position of the odor emitting object, and then flows toward the odor reducer.

According to this configuration, the odor is efficiently reduced by the combination of the device with the odor reducer.

A sixth aspect provides the spatial environment management system according to the fifth aspect, wherein the position acquisition unit is configured to acquire the information on the position of the odor reducer based on the image acquired by the acquisition unit.

According to this configuration, the position acquisition unit acquires the position of the odor reducer based on the image of the target space. Therefore, a user does not necessarily grasp the position of the odor reducer.

A seventh aspect provides the spatial environment management system according to the sixth aspect, wherein the odor reducer is an air cleaner including a deodorizing mechanism and an airflow producing mechanism. The position acquisition unit is further configured to acquire information on a position of an air suction port formed in the air cleaner, based on the image acquired by the acquisition unit. The control unit is configured to control the device such that air passes through or by the position of the odor emitting object, and then flows toward the suction port in the air cleaner.

According to this configuration, an odor substance is tend to be taken into the air cleaner as compared with a case where no consideration is given to the position of the suction port. The odor is thus reduced with ease.

An eighth aspect provides the spatial environment management system according to the seventh aspect, wherein when the determination unit determines that the odor emitting object is present in the target space, the control unit is further configured to control the air cleaner such that the air cleaner treats the odor.

According to this configuration, the control unit controls the operation of the air cleaner based on the determination by the determination unit rather than a result of detection by a sensor or the like of the air cleaner. The odor is tend to be rapidly reduced.

A ninth aspect provides the spatial environment management system according to any of the first to eighth aspects, wherein the control unit is configured to control at least one of an ON/OFF state of the device, a direction of air blown out from the device and a volume of air blown out from the device.

According to this configuration, the control unit appropriately operates the device on the odor by changing the ON/OFF state of the device, the direction of air from the device, and the volume of air from the device.

A tenth aspect provides the spatial environment management system according to any of the first to ninth aspects, wherein the device includes at least one of an air conditioning apparatus, an air cleaner, an electric fan, a ventilator and a deodorizing apparatus.

An eleventh aspect provides the spatial environment management system according to any of the first to tenth aspects, wherein the odor emitting object as a target of determination by the determination unit includes at least one of food, life and excrement.

A twelfth aspect provides the spatial environment management system according to any of the first to eleventh aspects, wherein when the determination unit determines that the odor emitting object is present in the target space, the control unit is configured to control the device in accordance with a content set based on action of a person on the odor emitting object.

According to this configuration, since the control unit controls the device based on the action of the person on the odor emitting object, the control unit appropriately operates the device on the odor.

A thirteenth aspect provides the spatial environment management system according to any of the first to twelfth aspects, wherein when the determination unit determines that a first object having an odor is present in the target space, the control unit is configured to commence first control on the device such that the device treats the odor. When the determination unit determines that the first object having the odor is absent from the target space after the commencement of the first control, the control unit is configured to implement second control different from the first control on the device such that the device treats the odor.

According to this configuration, the device further performs a predetermined operation even after the odor emitting object becomes absent from the target space. This configuration thus suppresses occurrence of a state in which the odor still remains in the target space although the odor emitting object becomes absent from the target space.

A fourteenth aspect provides the spatial environment management system according to the first aspect, wherein the device includes an air conditioning apparatus. When the determination unit determines that the odor emitting object is present in the target space, the control unit is configured to control the air conditioning apparatus such that the air conditioning apparatus performs one of a cooling operation, a dehumidifying operation, and a heating operation to treat the odor.

According to this configuration, since the cooling operation, the dehumidifying operation or the heating operation is utilized for treating the odor, the odor is removed from the target space with ease.

### BRIEF DESCRIPTION OF THE DRAWINGS

FIG. 1 is a schematic configuration diagram of a spatial environment management system according to a first embodiment.
FIG. 2 is a schematic block diagram of the spatial environment management system illustrated in FIG. 1.
FIG. 3 is a schematic block diagram of a control apparatus of the spatial environment management system illustrated in FIG. 1, the control apparatus including a controller of an air conditioning apparatus and a server.
FIG. 4 is a diagram of exemplary information stored in a pattern database of a storage unit of the control apparatus illustrated in FIG. 3.
FIG. 5 is a diagram of an air curtain operation by a ceiling-mounted indoor unit of the air conditioning apparatus in the spatial environment management system illustrated in FIG. 1.
FIG. 6 is an exemplary flowchart of odor control on a target space by the spatial environment management system illustrated in FIG. 1.
FIG. 7 is a schematic block diagram of a control apparatus of a spatial environment management system according to Modification 1E.
FIG. 8 is a schematic configuration diagram of a spatial environment management system according to a second embodiment.
FIG. 9 is a schematic block diagram of a control apparatus of the spatial environment management system illustrated in FIG. 8, the control apparatus including a controller of an air conditioning apparatus and a server.
FIG. 10 is an exemplary flowchart of odor control on a target space by the spatial environment management system illustrated in FIG. 8.

### DESCRIPTION OF EMBODIMENTS

A description will be given of a spatial environment management system according to an embodiment.

### <First Embodiment

A description will be given of a spatial environment management system 1 according to a first embodiment.

### (1) General Configuration

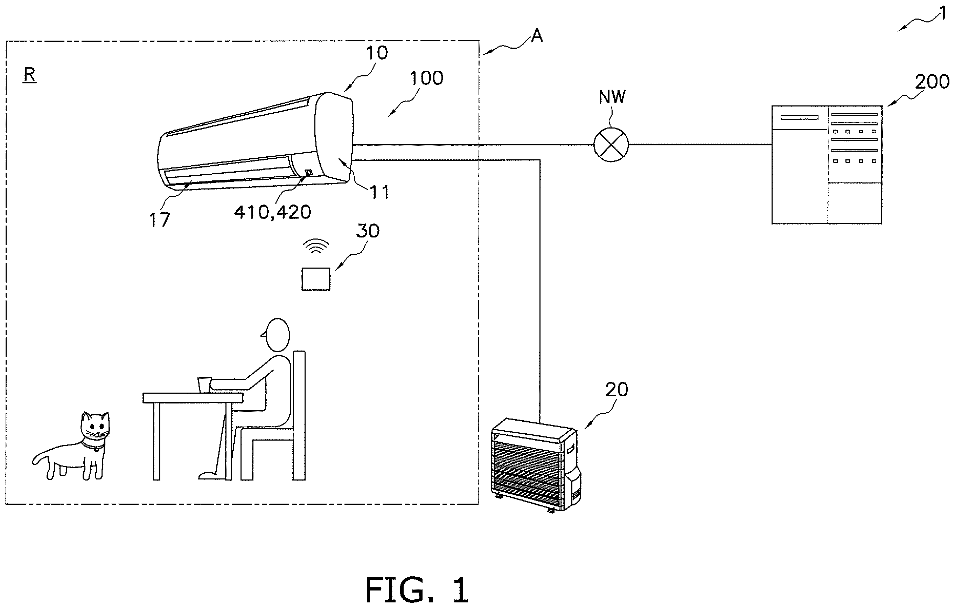

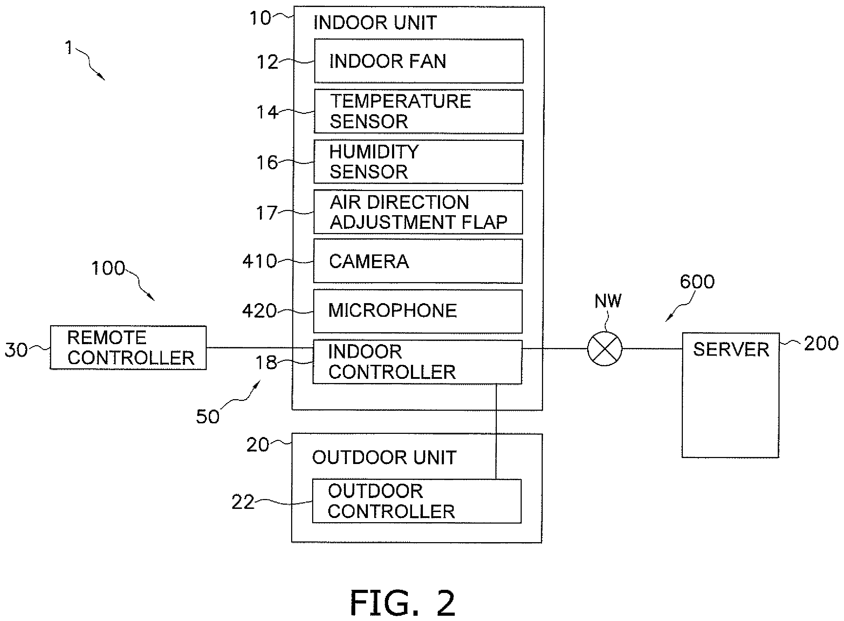

With reference to FIGS. 1 and 2, a description will be given of an outline of the spatial environment management system 1. FIG. 1 is a schematic configuration diagram of the spatial environment management system 1. FIG. 2 is a schematic block diagram of the spatial environment management system 1.

The spatial environment management system 1 includes a device including at least one of an airflow producing mechanism and a deodorizing mechanism for treating an odor in a target space R. In the following description, a device including at least one of an airflow producing mechanism and a deodorizing mechanism for treating an odor in the target space R is referred to as an "odor treatment device" in some cases for the sake of simplification. The spatial environment management system 1 determines whether an odor emitting object (hereinafter, referred to as an "odor source" in some cases for the sake of simplification) is present in the target space R. When determining that the odor source is present in the target space R, the spatial environment management system 1 controls the odor treatment device such that the odor treatment device treats the odor. The spatial environment management system 1 determines whether the odor source is present in the target space R, based on at least one of an image and a sound of the target space R.

In the first embodiment, the target space R is a room of a house in which a person lives. However, the target space R is not limited thereto. For example, the target space R may be a space in an office or a factory. Alternatively, the target space R may be a space in a commercial facility.

As used herein, a process for "treating an odor" includes various processes for a person in the target space R to be less susceptible to an influence of an odor. For example, the process for "treating an odor" may include a process of reducing the concentration of an odor substance in the air. The process for "treating an odor" may also include a process of avoiding the spread of an odor throughout the target space R. The process for "treating an odor" may also include a process of making an odor less likely to reach a person in the target space R. The process for "treating an odor" may also include a process of reducing an odor of an odor source itself.

In the first embodiment, examples of an odor source to be determined as to its presence by the spatial environment management system 1 may include, but not limited to, food (e.g., instant noodles and a hot pot dish that are apt to smell), life (e.g., a person and a pet), excrement, cigarettes (particularly, lit cigarettes), wet laundry hung indoors, and mold. The listed odor sources to be determined as to their presence by the spatial environment management system 1 are merely examples. The spatial environment management system 1 may alternatively determine presence of any odor source in addition to the listed odor sources. In addition, the spatial environment management system 1 does not necessary determine presence of some of or all of the listed odor sources.

A description will be given of a configuration of the spatial environment management system 1.

The spatial environment management system 1 mainly includes an air conditioning apparatus 100 and a server 200 connected to the air conditioning apparatus 100, specifically a controller 50 (to be described later) of the air conditioning apparatus 100 via a network NW so as to establish communications with the controller 50 (see FIGS. 1 and 2). The network NW used herein is the Internet; however, the network NW may be, for example, a wide area network or a local area network.

The air conditioning apparatus 100 is installed in a certain building (a house A) (see FIG. 1). The air conditioning apparatus 100 performs air conditioning in a room (a target space R) of the house A where the indoor unit 10 is placed (see FIG. 1).

The air conditioning apparatus 100 is an example of a device (an odor treatment device) including at least one of an airflow producing mechanism and a deodorizing mechanism. The indoor unit 10 of the air conditioning apparatus 100 includes an indoor fan 12 as an example of the airflow producing mechanism. However, the odor treatment device is not limited to the air conditioning apparatus 100. The spatial environment management system 1 may include, in place of the air conditioning apparatus 100, an electric fan or a ventilator (e.g., a ventilating fan) including an airflow producing mechanism, an air cleaner including an airflow producing mechanism and a deodorizing mechanism, or a deodorizing apparatus including at least a deodorizing mechanism, as an odor treatment device. The deodorizing mechanism is not limited to a deodorizing mechanism of a specific type. The deodorizing mechanism may be deodorizing mechanisms of publicly known various types such as a type of causing an adsorbent (e.g., active carbon) to adsorb an odor, a type of releasing ozone, and a type of releasing a deodorant.

The server 200 is a computer configured to execute various processes. The server 200 may be installed in any place. The server 200 is typically installed in a place different from the house A.

In FIG. 1, the server 200 is connected only to, but not limited to, the air conditioning apparatus 100 installed in the house A, via the network NW. The server 200 may be connected to air conditioning apparatuses (or other odor treatment devices) in multiple houses (or buildings other than houses) via the network NW. In other words, the server 200 may constitute the spatial environment management system 1 in conjunction with the air conditioning apparatus 100 installed in the house A, and may also constitute another spatial environment management system in conjunction with other odor treatment devices installed in different buildings. For the sake of simplifying the description, it is assumed herein that the server 200 is connected only to the air conditioning apparatus 100 in the house A via the network NW.

For the sake of simplifying the description, it is also assumed herein that the house A has one room (the target space R), one air conditioning apparatus 100 is installed in the house A to achieve air conditioning in the target space R, and the air conditioning apparatus 100 includes one indoor unit 10 placed in the target space R. However, the condition is not limited thereto. For example, the house A may have a plurality of rooms, and air conditioning apparatuses may be respectively installed in the rooms as an odor treatment device. For the sake of simplifying the description, it is also assumed herein that the air conditioning apparatus 100 includes one indoor unit 10 to be placed in the target space R. Alternatively, the air conditioning apparatus 100 may include at least two indoor units 10 to be placed in the target space R. An odor treatment device different in type from the air conditioning apparatus 100 may further be installed in the target space R in addition to the air conditioning apparatus 100 (i.e., the indoor unit 10).

The server 200 is one computer, but is not limited to a single computer. The server 200 may include a plurality of computers installed in the same place or respectively installed in different places, and connected to one another via a network.

### (2) Specific Configuration

With reference to FIGS. 1 to 5, a description will be further given of the air conditioning apparatus 100 and the server 200. In the following, a specific description will be given of a control apparatus 600 that includes the controller 50 of the air conditioning apparatus 100 and the server 200.

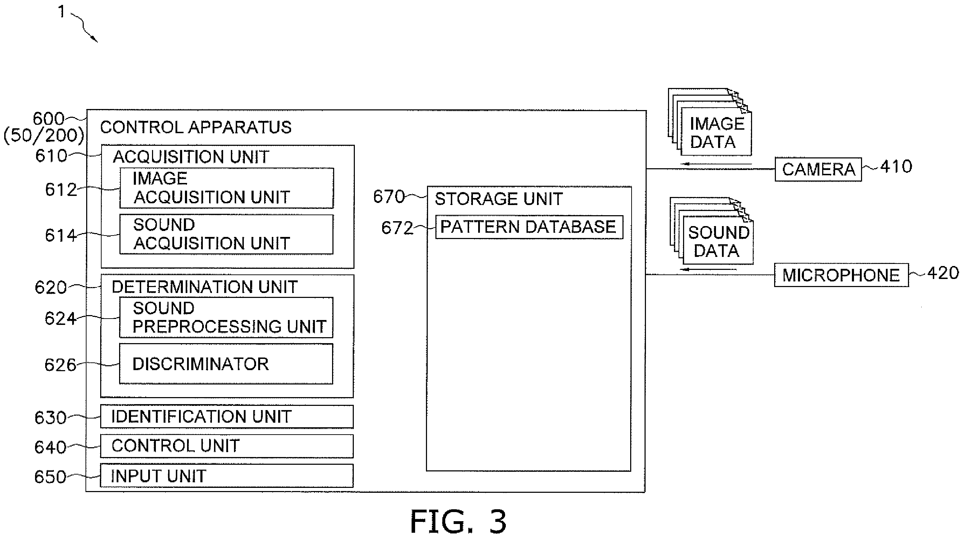

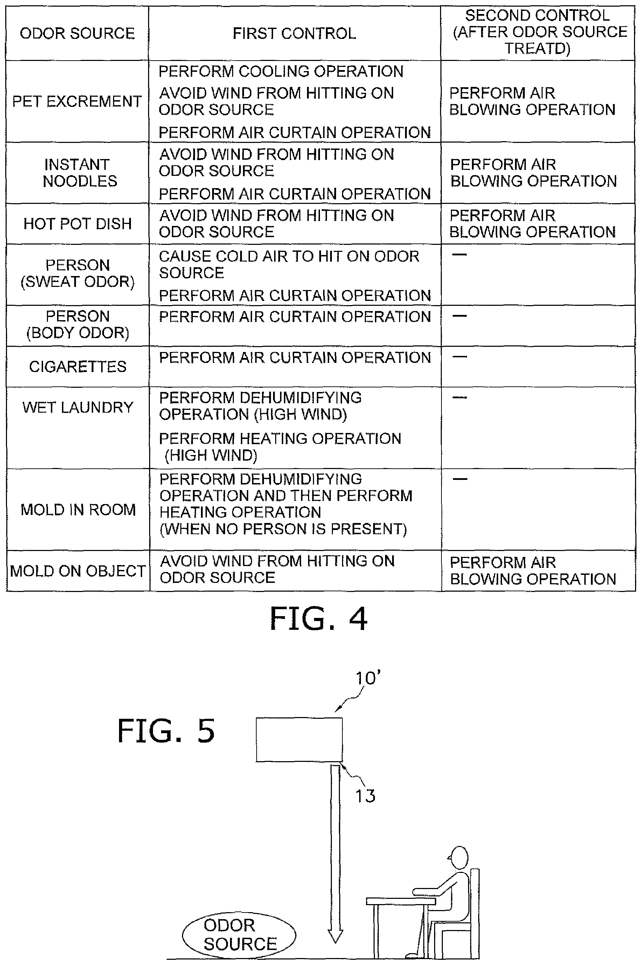

FIG. 3 is a schematic block diagram of the control apparatus 600 including the controller 50 of the air conditioning apparatus 100 and the server 200. FIG. 4 is a diagram of exemplary information stored in a pattern database 672 of a storage unit 670 in the control apparatus 600. FIG. 5 is a diagram of an air curtain operation by a ceiling-mounted indoor unit 10' of the air conditioning apparatus 100.

### (2-1) Air Conditioning Apparatus

The air conditioning apparatus 100 is configured to perform a cooling operation, a humidifying operation, and a heating operation in the target space R where the indoor unit 10 is placed. The air conditioning apparatus 100 has operating modes including a cooling operation mode, a dehumidifying operation mode, a heating operation mode, and an air blowing operation mode. According to another aspect, the air conditioning apparatus 100 may be, for example, a cooling-only apparatus. The air conditioning apparatus 100 performs operations such as a cooling operation and a heating operation using a vapor compression refrigeration cycle.

The air conditioning apparatus 100 mainly includes the indoor unit 10, an outdoor unit 20, a connection pipe (not illustrated) connecting the indoor unit 10 to the outdoor unit 20, and a remote controller 30 (see FIGS. 1 and 2). The indoor unit 10 illustrated in FIG. 1 is designed to be hung on a wall. Alternatively, the air conditioning apparatus 100 may include an indoor unit of any type such as an indoor unit designed to be mounted on a ceiling (e.g., an indoor unit designed to be embedded in a ceiling, an indoor unit designed to be suspended from a ceiling) or an indoor unit designed to be placed on a floor.

In the air conditioning apparatus 100, the indoor unit 10 is connected to the outdoor unit 20 via the connection pipe. Thereby, an indoor heat exchanger (not illustrated) of the indoor unit 10 as well as a compressor, an outdoor heat exchanger, an expansion valve, and the like (not illustrated) of the outdoor unit 20 are connected via pipes to form a refrigerant circuit. The air conditioning apparatus 100 causes a refrigerant to circulate through the refrigerant circuit such that the refrigerant circulating through the refrigerant circuit exchanges heat with air in the target space R. The air conditioning apparatus 100 thus achieves operations such as a cooling operation and a heating operation in the target space R where the indoor unit 10 is placed. Specifically, the air conditioning apparatus 100 achieves a cooling operation, a heating operation, and others in the target space R in a following manner. An indoor fan 12 (see FIG. 2) of the indoor unit 10 sucks air in the target space R into a housing 11 (see FIG. 1) of the indoor unit 10 and supplies it to the indoor heat exchanger. Then, the air being subjected to heat exchange with the refrigerant flowing through the indoor heat exchanger is blown out the air toward the target space R. The operation principle of the air conditioning apparatus 100 using the vapor compression refrigeration cycle is publicly known; therefore, the description thereof will not be given here.

The indoor unit 10 includes the indoor heat exchanger (not illustrated), the indoor fan 12, the housing 11 accommodating therein the indoor heat exchanger and the indoor fan 12, a temperature sensor 14 configured to measure a temperature of the target space R, a humidity sensor 16 configured to measure a humidity of the target space R, an air direction adjustment flap 17, and an indoor controller 18 (see FIGS. 1 and 2). The indoor fan 12 is an example of the airflow producing mechanism. The indoor fan 12 may be, but not limited to, a fan configured to blow out a variable volume of air. The air direction adjustment flap 17 is disposed on a blow-out port through which the indoor unit 10 blows out air. The air direction adjustment flap 17 is driven by a motor (not illustrated) to adjust a direction of air to be blown out through the blow-out port. The indoor controller 18 is, for example, a micro control unit (MCU) mainly including a central processing unit (CPU), a memory, an input-output port, and the like. The indoor unit 10 is equipped with a camera 410 and a microphone 420. The camera 410 and the microphone 420 will be described later.

The outdoor unit 20 mainly includes a compressor, an outdoor heat exchanger, an expansion valve, and an outdoor fan (not illustrated) as well as an outdoor controller 22. The outdoor controller 22 includes, for example, an MCU mainly including a CPU, a memory, an input-output port, and the like.

The remote controller 30 is a device configured to receive the inputting of various instructions and various kinds of information from a user of the air conditioning apparatus 100. The remote controller 30 includes an MCU mainly including a CPU, a memory, an input-output port, and the like. The remote controller 30 may include a display unit (not illustrated) configured to display thereon, for example, a current operating state of the air conditioning apparatus 100.

The indoor controller 18, the outdoor controller 22, and the remote controller 30 are connected to establish mutual communications, and constitute the controller 50 as a whole. The controller 50 controls the operation of the air conditioning apparatus 100. The controller 50 executes a program stored in the memory, thereby controlling the operation of components for air conditioning in the air conditioning apparatus 100, based on instructions input to the remote controller 30 by the user and measured values of sensors in the respective units of the air conditioning apparatus 100. The sensors in the respective units of the air conditioning apparatus 100 include the temperature sensor 14 and the humidity sensor 16. Examples of the components for air conditioning in the air conditioning apparatus 100 whose operation is controlled by the controller 50 include: the compressor, the expansion valve, and the outdoor fan (not illustrated) of the outdoor unit 20; and the indoor fan 12 and air direction adjustment flap 17 of the indoor unit 10. The control by the controller 50 on the operation of the components for air conditioning is typically known; therefore, the description thereof will not be given here. The control by the control apparatus 600 on the air conditioning apparatus 100 for treating an odor in the target space R (the control on the various components constituting the air conditioning apparatus 100) will be described later.

The controller 50 is connected to the network NW via a modem or an optical network unit, a router, and others (not illustrated), so that the controller 50 is connected to the server 200 so as to establish communications with the server 200. The controller 50 constitutes the control apparatus 600 in conjunction with the server 200 (see FIG. 3).

The control apparatus 600 controls the operation of the air conditioning apparatus (the odor treatment device) 100 in the spatial environment management system 1. In summary, the control apparatus 600 determines whether an odor source is present in the target space R, based on at least one of an image and a sound of the target space R. When determining that an odor source is present in the target space R, the control apparatus 600 controls the air conditioning apparatus 100 such that the air conditioning apparatus 100 treats an odor. The control apparatus 600 will be described in detail later.

### (2-1-1) Camera

The camera 410 is an apparatus configured to capture an image of the target space R. Preferably, the camera 410 is an apparatus configured to capture a moving image. However, the camera 410 may be an apparatus configured to capture a still image in a case where a determination unit 620 (to be described later) uses a still image in order to make a determination. Preferably, the camera 410 is configured to capture a color image. In a case where, for example, hue information is not used, the camera 410 may be configured to capture a monochrome image. The camera 410 is connected to the control apparatus 600 so as to establish communications with the control apparatus 600. The camera 410 transmits a captured image (image data) to the control apparatus 600.

The camera 410 is disposed on, for example, an outer surface of a front panel of the housing 11 of the indoor unit 10 (see FIG. 1). The camera 410 is not necessarily disposed on the outer surface of the front panel of the housing 11. The position of the camera 410 may be appropriately selected. For example, the camera 410 may be disposed on a lower surface of the housing 11. Alternatively, the camera 410 may be disposed, for example, at a position adjacent to the housing 11 rather than on the housing 11. Preferably, the camera 410 is disposed at a position where the camera 410 captures an image of a wide area of the target space R with ease.

According to another embodiment, the camera 410 may be an apparatus provided independently of the indoor unit 10 and disposed at a position distant from the housing 11. Also in this case, preferably, the camera 410 is connected to the control apparatus 600 so as to establish communications with the control apparatus 600, and is configured to transmit image data to the control apparatus 600.

In the first embodiment, the number of cameras 410 is one. However, the number of cameras 410 is not limited to one. The indoor unit 10 may be equipped with a plurality of cameras 410.

### (2-1-2) Microphone

The microphone 420 is an apparatus configured to convert a sound of the target space R into an electric signal, thereby acquiring the sound in the form of the electric signal. The microphone 420 preferably includes a plurality of microphone elements in order to identify a direction from which a sound is generated (e.g., a direction from which a voice is originated). The microphone 420 is connected to the control apparatus 600 so as to establish communications with the control apparatus 600. The microphone 420 transmits an acquired sound (sound data) to the control apparatus 600.

The microphone 420 is disposed on, for example, the outer surface of the front panel of the housing 11 of the indoor unit 10 at a position near the camera 410 (see

FIG. 1). The microphone 420 is not necessarily disposed on the outer surface of the front panel of the housing 11. The position of the microphone 420 may be appropriately selected. For example, the microphone 420 may be disposed at a position distant from the camera 410. Alternatively, the microphone 420 may be disposed on the lower surface of the housing 11. Alternatively, the microphone 420 may be disposed, for example, at a position adjacent to the housing 11 rather than on the housing 11. Preferably, the microphone 420 is disposed at a position where the microphone 420 acquires a sound of the target space R with ease.

According to another embodiment, the microphone 420 may be an apparatus provided independently of the indoor unit 10 and disposed at a position distant from the housing 11. Also in this case, preferably, the microphone 420 is connected to the control apparatus 600 so as to establish communications with the control apparatus 600, and is configured to transmit sound data to the control apparatus 600.

In the first embodiment, the microphone 420 (a group of microphone elements) is disposed on a single place. Alternatively, the microphone elements may be respectively disposed on different places.

### (2-2) Server

The server 200 is a computer connected to the controller 50 of the air conditioning apparatus 100 via the network NW so as to establish communications with the controller 50 and constituting the control apparatus 600 in conjunction with the controller 50. The server 200 mainly includes a CPU, a random access memory, a read only memory (ROM), and an external storage device such as a hard disk. The server 200 executes various programs stored in the ROM, the external storage device, and the like, thereby executing various processes as the control apparatus 600 in conjunction with the controller 50.

### (2-3) Control Apparatus

The control apparatus 600 includes the controller 50 of the air conditioning apparatus 100 and the server 200 with the controller 50 and the server 200 connected via the network NW so as to establish communications with each other.

The control apparatus 600 includes as its functional unit an acquisition unit 610, a determination unit 620, an identification unit 630, a control unit 640, an input unit 650, and a storage unit 670 (see FIG. 3).

In the first embodiment, for example, the server 200 has functions as the acquisition unit 610, the determination unit 620, and the identification unit 630, the remote controller 30 of the controller 50 has a function as the input unit 650, and the controller 50 has a function as the control unit 640. The controller 50 includes the pattern database 672 of the storage unit 670. The control apparatus 600 is only required to function as the acquisition unit 610, the determination unit 620, the identification unit 630, the control unit 640, the input unit 650, and the storage unit 670 as a whole. These functions may be appropriately allocated to the respective units of the controller 50 and server 200.

The spatial environment management system 1 may include an odor sensor (not illustrated). The control apparatus 600 may also control the air conditioning apparatus 100 based on a result of detection of an odor by the odor sensor (not illustrated). The first embodiment does not describe the control on the air conditioning apparatus 100 based on a result of detection by the odor sensor.

### (2-3-1) Acquisition Unit

The acquisition unit 610 acquires at least one of an image of the target space R and a sound of the target space R. In the first embodiment, the acquisition unit 610 acquires both of an image and a sound of the target space R.

The acquisition unit 610 includes an image acquisition unit 612 configured to acquire an image (image data) of the target space R transmitted from the camera 410. The acquisition unit 610 also includes a sound acquisition unit 614 configured to acquire a sound (sound data) of the target space R transmitted from the microphone 420. The image and sound of the target space R acquired by the acquisition unit 610 are stored in the storage unit 670, and are used for, for example, processes to be executed by the determination unit 620 and the identification unit 630 as will be described later.

### (2-3-2) Determination Unit

The determination unit 620 determines whether an odor source is present in the target space R, based on at least one of an image and a sound of the target space R acquired by the acquisition unit 610. The determination unit 620 also determines a type of an odor source in the target space R, based on at least one of an image and a sound of the target space R acquired by the acquisition unit 610. Examples of an odor source to be determined by the determination unit 620 as to its presence in the target space R may include food, life, pet excrement, cigarettes (particularly, lit cigarettes), wet laundry hung indoors, and mold. Examples of the food may include, but not limited to, instant noodles and a hot pot dish that are apt to smell. Examples of the life may include, but not limited to, a person and a pet. The determination unit 620 may determine a type of an odor source by further classifying the odor source. For example, the determination unit 620 may determine a "person" as an odor source while classifying the "person" into, for example, a "person having a sweat odor (a person who is sweating)" and a "person having a body odor (a person who has returned from the outside)".

The determination unit 620 includes a sound preprocessing unit 624 and a discriminator 626 (see FIG. 3).

The determination unit 620 determines, using the learned discriminator 626, whether an odor source is present in the target space R and what type of the odor source is, based on at least one of image data and sound data acquired by the acquisition unit 610. Learning will be described later.

The sound preprocessing unit 624 detects a target sound section, as preprocessing for sound data acquired by the acquisition unit 610, to discriminate, as to sound data containing a target sound and noise, between a section where the target sound is present and a section different from that section and to extract the section where the target sound is present from the sound data. The target sound is mainly a sound which a person or a pet in the target space R makes. In addition, the sound preprocessing unit 624 extracts a sound feature amount from the target sound.

### (2-3-2-1) Sound Preprocessing Unit

The sound preprocessing unit 624 detects a target sound section to discriminate, as to sound data containing a target sound and noise, a section where the target sound is present and a section different from that section, and to extract the section where the target sound is present from the sound data. For example, in a case where a volume equal to or more than a predetermined volume lasts for a predetermined time or more in sound data, the sound preprocessing unit 624 detects this section as a target sound section. However, the method of detecting the target sound section is not limited thereto, and publicly known various methods may be used. In addition, the sound preprocessing unit 624 extracts a sound feature amount from the target sound while referring to, for example, an acoustic model (not illustrated). Examples of the sound feature amount may include, but not limited to, a volume, a tempo, a tone, and the meaning of a voice. The sound preprocessing unit 624 also refers to a word pronunciation model, a language model, and the like (not illustrated) in addition to the acoustic model, converts a voice into a text, and recognizes the meaning of the voice. Publicly known various methods may be used for the voice recognition by the sound preprocessing unit 624.

### (2-3-2-2) Discriminator

The discriminator 626 is subjected to a learning process so as to output, as a result of determination, presence or absence of an odor source in the target space R and a type of an odor source in the target space R (in the case where the odor source is present in the target space R) when receiving information on at least one of an image of the target space R and a sound of the target space R (particularly a sound feature amount). In the first embodiment, the discriminator 626 is subjected to a learning process based on machine learning. The machine learning means a technique and a method, which are not rule-based (i.e. rules for determination being not given to the computer in advance), for a computer to learn about given information and autonomously find rules for determination.

In a case where the discriminator 626 needs to discriminate different inputs, the discriminator 626 may include a plurality of the discriminators. For example, in a case where the discriminator 626 needs to determine presence or absence and a type of a certain odor source only from information on an image, needs to determine presence or absence and a type of another odor source only from information on a sound, and needs to determine presence or absence and a type of still another odor source from both information on an image and information on a sound, the discriminator 626 may include a plurality of the discriminators configured to discriminate different inputs, respectively.

The discriminator 626 has already been learned based on, for example, supervised learning. The supervised learning means a method of machine learning for the discriminator 626 to learn about data (training data) in which an input is associated with an output as a correct answer (e.g., a type of an odor source) corresponding to the input.

For example, the discriminator 626 during the learning receives, as training data, multiple pieces of data in which images of a determination target odor source captured at various angles and in various sizes are associated with a type of the odor source in the images. The discriminator 626 during the learning also receives, as training data, multiple pieces of data in which sound feature amounts of sounds corresponding to odor sources (e.g., a sound which a person makes while sucking in instant noodles, a sound which a pet makes while excreting) are associated with types of the odor sources corresponding to the sounds. In addition to these pieces of data or in place of these pieces of data, the discriminator 626 during the learning may receive, as training data, multiple pieces of data in which images of a determination target odor source captured at various angles and in various sizes, various sounds corresponding to the odor source, and a type of the odor source are associated with one another.

A learning algorithm for use in the discriminator 626 is, for example, a neural network (including deep learning). Alternatively, other publicly known machine learning algorithms (e.g., a support vector machine) may be used. In the case where the discriminator 626 includes the plurality of discriminators for discriminating different inputs as described above or in a case where a process is divided into multiple processes and different discriminators respectively execute the divided processes as will be described later, different learning algorithms may be used for the learning on the respective discriminators.

When receiving at least one of an image of the target space R and a sound feature amount extracted by the sound preprocessing unit 624, the determination unit 620 determines whether an odor source is present in the target space R, using the learned discriminator 626. For example, the determination unit 620 determines whether a region where an odor source of a certain type comes out is present in the image of the target space R. For example, the determination unit 620 determines whether the region where the odor source of the certain type comes out is present, based on a shape, a hue, and the like appearing in the image of the target space R. For example, the determination unit 620 also determines whether the sound feature amount extracted by the sound preprocessing unit 624 includes a sound feature amount at the time when the odor source of the certain type is present in the target space R.

The determination unit 620 may determine whether an odor source is present in the target space R, based on at least one of an image of the target space R and a sound of the target space R and other information other than the image of the target space R and the sound of the target space R.

For example, the determination unit 620 may determine a state in which a person has entered the target space R (e.g., a state in which a person has returned the house A), based on an image of the target space R, and may determine whether the person in the target space R (e.g., the person who has returned the house A) is sweating, using information on an outdoor temperature measured by a temperature sensor configured to measure an outdoor temperature.

### (2-3-3) Identification Unit

The identification unit 630 identifies, when the determination unit 620 determines that an odor source is present in the target space R, a position of the odor source in the target space R based on at least one of an image and a sound of the target space R acquired by the acquisition unit 610.

For example, when the determination unit 620 determines that a region where the odor source comes out (referred to as an odor source region) is present in the image of the target space R, the identification unit 630 identifies a position of the odor source in an actual space, from a position of the odor source region in the image of the target space R, using a calculation formula for associating the position of the odor source region in the image of the target space R with the position of the odor source in the actual space, a database storing the correlation, and/or others. Preferably, the identification unit 630 identifies a position of the odor source relative to the indoor unit 10 of the air conditioning apparatus 100.

For example, when the determination unit 620 determines that a sound feature amount extracted by the sound preprocessing unit 624 includes a sound feature amount corresponding to the presence of the odor source, the identification unit 630 ascertains from which direction a sound as the original data of the sound feature amount is emitted, and identifies the direction as the position (direction) of the odor source. For example, in a case where the sound feature amount extracted by the sound preprocessing unit 624 includes a sound feature amount of a sound which a person makes while sucking in instant noodles, the identification unit 630 ascertains from which direction the sound as the original data of the sound feature amount is emitted, and identifies the direction as the position of the odor source. More specifically, the identification unit 630 obtains information from the microphone 420, and ascertains from which direction the sound as the original data is emitted with respect to the microphone 420. As another method, when the determination unit 620 determines that the sound feature amount extracted by the sound preprocessing unit 624 includes a sound feature amount corresponding to the presence of an odor source, the identification unit 630 may ascertain where a person or a pet (which is considered to make a sound) is, from an image of the target space R captured at the time when a sound as the original data of the sound feature amount is collected, and identify the position of the person or the pet as a position of the odor source.

The method of identifying the position of the odor source by the identification unit 630 is not limited to the method described above, and various methods may be applied thereto.

### (2-3-4) Control Unit

The control unit 640 controls the odor treatment device (the air conditioning apparatus 100 in the first embodiment) such that the air conditioning apparatus 100 treats an odor when the determination unit 620 determines that an odor source is present in the target space R.

Preferably, the control unit 640 has a plurality of control patterns for the air conditioning apparatus 100, which can be used when the determination unit 620 determines that an odor source is present in the target space R. In other words, preferably, the control unit 640 operates the air conditioning apparatus 100 in accordance with various control contents (at least two kinds of control contents) when the determination unit 620 determines that an odor source is present in the target space R. The term "the control patterns for the air conditioning apparatus 100" used herein has substantially the same meaning as the term "the control contents of the air conditioning apparatus 100". Preferably, when the determination unit 620 determines that an odor source is present in the target space R, the control unit 640 controls the air conditioning apparatus 100 in accordance with a content (a control pattern) set based on a type of the odor source determined by the determination unit 620.

Specifically, when the determination unit 620 determines that an odor source is present in the target space R, the control unit 640 refers to the pattern database 672 of the storage unit 670. The control unit 640 sets a control pattern corresponding to a type of the odor source determined by the determination unit 620 to be present in the target space R, based on the information stored in the pattern database 672 of the storage unit 670, and controls the air conditioning apparatus 100 in accordance with the set control pattern.

The pattern database 672 stores, for example, a list of information such as information illustrated in FIG. 4. In the list illustrated in FIG. 4, a type of an odor source is associated with a corresponding control pattern for the air conditioning apparatus 100. As illustrated in the list of FIG. 4, there are a type of an odor source associated with one control pattern (first control) and a type of an odor source associated with two control patterns (first control, second control). The first control is a control pattern to be implemented when the determination unit 620 determines that an odor source is present in the target space R. The second control is a control pattern to be implemented when the determination unit 620 determines that the first odor source becomes absent from the target space R, after the control unit 640 commences the first control on the air conditioning apparatus 100 (i.e., after the control unit 640 commences control on the air conditioning apparatus 100 in accordance with the content of the first control) in a case when the determination unit 620 determines that an odor source (referred to as a first odor source) is present in the target space R.

With reference to FIG. 4, a description will be given of types of odor sources and control patterns corresponding to the types of the odor sources. The list of FIG. 4 and the following description are merely examples of a method for controlling the air conditioning apparatus 100 by the control unit 640. The control unit 640 may control the operation of the air conditioning apparatus 100 in accordance with control contents different from those to be described below.

### <Example 1> Case where odor source is pet excrement

As illustrated in FIG. 4, two control patterns, that is, first control and second control are prepared for pet excrement.

When the determination unit 620 determines that pet excrement is present in the target space R, the control unit 640 commences the first control on the air conditioning apparatus 100 such that the air conditioning apparatus 100 treats the odor. In other words, when the determination unit 620 determines that pet excrement is present in the target space R, the control unit 640 commences control on the air conditioning apparatus 100 in accordance with the control pattern for (i.e., the control content of) the first control such that the air conditioning apparatus 100 treats the odor. Specifically, when the determination unit 620 determines that pet excrement is present in the target space R, the control unit 640 controls the air conditioning apparatus 100 such that the air conditioning apparatus 100 performs a cooling operation to treat the odor. For example, the control unit 640 controls the air conditioning apparatus 100 at a standstill such that the air conditioning apparatus 100 commences the cooling operation. As the first control, the control unit 640 controls the air conditioning apparatus 100 in accordance with the content set based on the position of the pet excrement identified by the identification unit 630. Specifically, the control unit 640 adjusts a direction of air blown out from the indoor unit 10 (i.e., controls the operation of the air direction adjustment flap 17) so as to avoid the wind generated by the indoor unit 10 from directly hitting on the pet excrement. In a case where the indoor unit of the air conditioning apparatus 100 is a ceiling-mounted (e.g., ceiling-embedded) indoor unit 10' as illustrated in FIG. 5 and can blow out air vertically downward or almost vertically downward, the control unit 640 controls the air conditioning apparatus 100 such that the indoor unit 10' performs an air curtain operation. In other words, the control unit 640 controls an air direction adjustment flap (not illustrated in FIG. 5) such that the indoor unit 10' blows out air vertically downward or almost vertically downward through a blow-out port 13 (see FIG. 5). The indoor unit 10' performs the air curtain operation in a case where an odor source (e.g., pet excrement) and a person are located on opposite sides of the target space R across an imaginary plane extending vertically downward from the air blow-out port 13 in the indoor unit 10'. The indoor unit 10' performs the air curtain operation to form an air wall between the odor source and the person such that the odor of the odor source hardly reaches the person. Whether a person is present in the target space R and where the person is in the target space R are ascertained in such a manner that the determination unit 620 determines the presence of the person and the identification unit 630 identifies the position of the person. In place of the method, the position of the person may be identified using another detector (e.g., an infrared camera).

When the determination unit 620 determines that the pet excrement becomes absent from the target space R (i.e., when the pet excrement was removed) after the commencement of the first control, the control unit 640 implements the second control on the air conditioning apparatus 100 such that the air conditioning apparatus 100 treats the odor. In other words, when the determination unit 620 determines that the pet excrement is absent from the target space R after the commencement of the first control, the control unit 640 commences control on the air conditioning apparatus 100 in accordance with the control pattern for (i.e., the control content of) the second control such that the air conditioning apparatus 100 treats the odor. The second control is different from the first control. In other words, the control content of the second control is different from the control content of the first control. By the second control, the air conditioning apparatus 100 blows air toward a place where the pet excrement was present before being removed. Specifically, the control unit 640 changes the cooling operation to an air blowing operation, and adjusts a direction of air blown out from the indoor unit 10 (i.e., controls the operation of the air direction adjustment flap 17) so as to direct wind generated by the indoor unit 10 to the place where the pet excrement was present. The air blowing operation is an operation of driving only the indoor fan 12 without causing the refrigerant to circulate through the refrigerant circuit in the air conditioning apparatus 100. According to another aspect, the control unit 640 may adjust the direction of the air blown out from the indoor unit 10 such that the indoor unit 10 blows out air toward the place where the pet excrement was present, while maintaining the cooling operation.

### <Example 2> Case where odor source is instant noodles

As illustrated in FIG. 4, two control patterns, that is, first control and second control are prepared for instant noodles.

The content of the first control in the case where an odor source is instant noodles is similar to the content of the first control in the case where an odor source is pet excrement, except that the air conditioning apparatus 100 does not perform the cooling operation (i.e., the air conditioning apparatus 100 does not perform the cooling operation by changing the operating mode); therefore, the description thereof will not be given here. In addition, the content of the second control in the case where an odor source is instant noodles is similar to the content of the second control in the case where an odor source is pet excrement; therefore, the description thereof will not be given here.

According to another aspect, when the determination unit 620 determines that instant noodles is present in the target space R, the control unit 640 may control the air conditioning apparatus 100 in accordance with the content set based on the action of a person on the odor emitting object. It is assumed herein that the discriminator 626 of the determination unit 620 learns about the action of a person so as to determine how the person acts, from an image of the person in the target space R. Specifically, when the determination unit 620 determines that instant noodles is present in the target space R and also determines that a person located near the instant noodles is eating, the control unit 640 adjusts a direction of air blown out from the indoor unit 10 so as avoid wind generated by the indoor unit 10 from directly hitting on the instant noodles. When the determination unit 620 determines that the person located near the instant noodles finished eating, the control unit 640 adjusts the direction of air blown out from the indoor unit 10 so as to cause the wind generated by the indoor unit 10 to directly hit on the instant noodles.

### <Example 3> Case where odor source is person who is sweating

As illustrated in FIG. 4, one control pattern, that is, first control is prepared for a person who is sweating.

When the determination unit 620 determines that a person who is sweating is present in the target space R, the control unit 640 commences the first control on the air conditioning apparatus 100 such that the air conditioning apparatus 100 treats the odor. In other words, when the determination unit 620 determines that a person who is sweating is present in the target space R, the control unit 640 commences control on the air conditioning apparatus 100 in accordance with the control pattern for (i.e., the control content of) the first control such that the air conditioning apparatus 100 treats the odor. Specifically, when the determination unit 620 determines that the person who is sweating is present in the target space R, the control unit 640 controls the air conditioning apparatus 100 such that the air conditioning apparatus 100 performs the cooling operation to treat the odor, and adjusts a direction of air blown out from the indoor unit 10 so as to cause wind generated by the indoor unit 10 to directly hit on the person who is sweating. In the case where the indoor unit of the air conditioning apparatus 100 is the ceiling-mounted indoor unit 10' as illustrated in FIG. 5 and blows out air vertically downward or almost vertically downward, the control unit 640 controls the air conditioning apparatus 100 such that the indoor unit 10' performs the air curtain operation.

Since second control is not prepared for a person who is sweating as an odor source as illustrated in FIG. 4, the control unit 640 does not implement further control for treating the odor when the person who is sweating left the target space R.

### <Example 4> Case where odor source is wet laundry hung indoors

As illustrated in FIG. 4, one control pattern, that is, first control is prepared for wet laundry hung indoors.

When the determination unit 620 determines that wet laundry is present in the target space R, the control unit 640 controls the air conditioning apparatus 100 such that the air conditioning apparatus 100 performs a dehumidifying operation or a heating operation with a volume of air from the indoor fan 12 adjusted to a larger value (e.g., the maximum value). The control unit 640 may previously determine which of the dehumidifying operation and the heating operation the air conditioning apparatus 100 performs. Alternatively, the control unit 640 may previously determine which of the dehumidifying operation and the heating operation the air conditioning apparatus 100 performs, based on, for example, a temperature of the target space R measured by the temperature sensor 14.

Since second control is not prepared for wet laundry hung indoors as an odor source as illustrated in FIG. 4, the control unit 640 does not implement further control for treating the odor when the laundry was absent from the target space R.

### <Example 5> Case where odor source is mold in room

As illustrated in FIG. 4, one control pattern, that is, first control is prepared for mold in a room.

When the determination unit 620 determines that mold is on, for example, a wall of the target space R and also determines that no person is present in the target space R (i.e., when the determination unit 620 determines that no person is present in the target space R or when another detector (e.g., an infrared camera) determines that no person is present in the target space R), the control unit 640 controls the air conditioning apparatus 100 such that the air conditioning apparatus 100 performs the dehumidifying operation, and then performs the heating operation. It is assumed herein that a time for the dehumidifying operation and a time for the heating operation are set in advance.

Since second control is not prepared for mold in a room as an odor source as illustrated in FIG. 4, the control unit 640 does not implement further control for treating the odor when the mold was absent from the target space R.

### <Example 6> Case where odor source is mold on object

As illustrated in FIG. 4, two control patterns, that is, first control and second control are prepared for mold on an object (i.e., an object to which mold adheres).

When the determination unit 620 determines that an object to which mold adheres is present in the target space R, the control unit 640 commences the first control on the air conditioning apparatus 100 such that the air conditioning apparatus 100 treats the odor. In other words, when the determination unit 620 determines that an object to which mold adheres is present in the target space R, the control unit 640 commences control on the air conditioning apparatus 100 in accordance with the control pattern for (i.e., the control content of) the first control such that the air conditioning apparatus 100 treats the odor. Specifically, when the determination unit 620 determines that an object to which mold adheres is present in the target space R, the control unit 640 adjusts a direction of air blown out from the indoor unit 10 so as to avoid the wind generated by the indoor unit 10 from directly hitting on the mold on the object. In place of the control content, when the determination unit 620 determines that an object to which mold adheres is present in the target space R, the control unit 640 may stop the air conditioning apparatus 100 so as to avoid wind generated by the indoor unit 10 from hitting on the mold on the object.

The content of the second control in the case where an odor source is mold on an object is similar to the content of the second control in the case where an odor source is pet excrement; therefore, the description thereof will not be given here.

### (2-3-5) Input Unit

The input unit 650 is a functional unit configured to accept an instruction on the air conditioning apparatus 100 from the user. For example, the input unit 650 receives instructions for settings of an ON or OFF state and the operating modes (e.g., the cooling operation, the heating operation, the dehumidifying operation, the air blowing operation) of the air conditioning apparatus 100 and instructions for settings of a volume and a direction of air blown out from the indoor unit 10. When it is determined that an odor source is present in the target space R, the input unit 650 receives an instruction as to whether the air conditioning apparatus 100 performs an operation for treating the odor (referred to as an odor treating operation). In the spatial environment management system 1, when the input unit 650 receives an instruction for performing the odor treating operation, the control unit 640 controls the operation of the air conditioning apparatus 100, based on a result of determination by the determination unit 620. A description on the operation of the spatial environment management system 1 in the case where the odor treating operation is not performed will not be given here.

Preferably, in the control apparatus 600, information stored in the pattern database 672 is rewritable based on a user input to the input unit 650. In other words, preferably, when it is determined that an odor source of a certain type is present in the target space R, the control apparatus 600 is configured to change a control pattern for controlling the operation of the air conditioning apparatus 100 by the control unit 640, in accordance with a user input. In the control apparatus 600, all the pieces of information stored in the pattern database 672 are not necessarily rewritten. For example, only the contents of first control illustrated in FIG. 4 may be rewritten.

### (2-3-6) Storage Unit

The storage unit 670 stores various kinds of information for use in the control apparatus 600. For example, the storage unit 670 stores an image and a sound of the target space R acquired by the acquisition unit 610.

The storage unit 670 has the pattern database 672 as an information storage region. The information stored in the pattern database 672 has already been described above; therefore; the description thereof will not be given here.

The pattern database 672 may store default data in advance. According to another aspect, the pattern database 672 stores no default data, and the control apparatus 600 may issue to the user a request to input information to the pattern database 672 at the time when the user starts to utilize the spatial environment management system 1.

### (3) Odor Control on Target Space

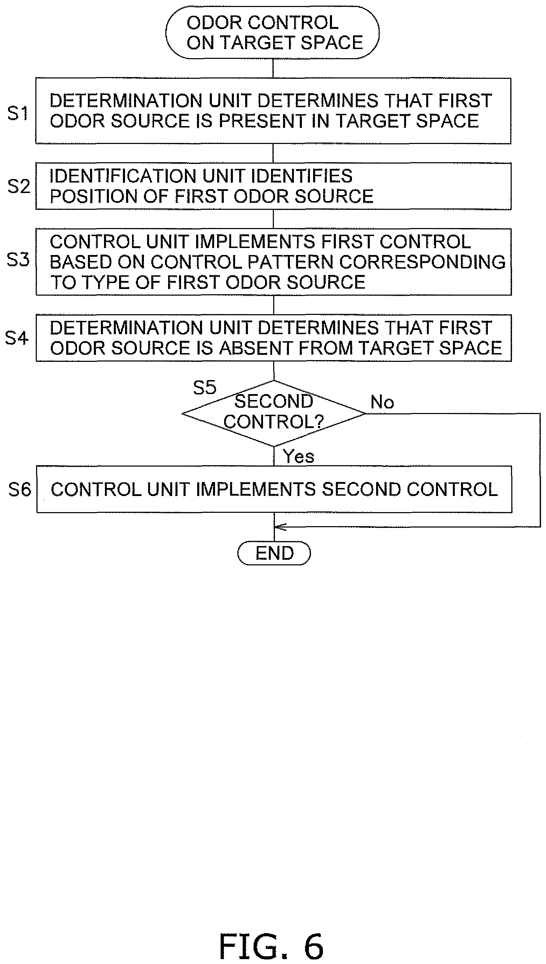

With reference to FIG. 6, a description will be given of odor control on the target space R by the spatial environment management system 1. The flowchart to be described below is merely an exemplary control flow, and therefore does not intend to limit a control flow.

It is assumed herein that the determination unit 620 determines whether an odor source is present in the target space R, and also determines a type of the odor source (when the odor source is present in the target space R), based on an image and a sound of the target space R acquired by the acquisition unit 610 (e.g., an image captured by the camera 410 and a sound collected by the microphone 420 in a preceding predetermined period (e.g., 10 second)) every predetermined time. The predetermined time is not limited. Preferably, the predetermined time is relatively short (e.g., one minute).

It is assumed that at a certain time (i.e., in step S1), the determination unit 620 determines that an odor source of a certain type (referred to as a first odor source) is present in the target space R, based on at least one of an image or a sound of the target space R acquired by the acquisition unit 610. In this case, the processing of the odor control on the target space R proceeds to step S2. It is assumed that after determining that the first odor source is present in the target space R, the determination unit 620 continuously determines whether the odor source is present in the target space R (whether the first odor source determined as being present in the target space R become absent from the target space R) every predetermined time (e.g., every one minute), based on at least one of an image or a sound of the target space R acquired by the acquisition unit 610.

In step S2, the identification unit 630 identifies a position of the first odor source. Preferably, the identification unit 630 identifies a positional relationship between the first odor source and the indoor unit 10 of the air conditioning apparatus 100.

Next, the control unit 640 refers to the pattern database 672 of the storage unit 670 to ascertain a control pattern (i.e., first control) corresponding to the type of the odor source (i.e., the first odor source) determined by the determination unit 620. The control unit 640 commences the first control on the air conditioning apparatus 100 such that the air conditioning apparatus 100 treats the odor. In other words, the control unit 640 controls the air conditioning apparatus 100, based on the content of the ascertained control pattern (i.e., the first control) (step S3).

For example, in the case where the pattern database 672 of the storage unit 670 stores the list illustrated in FIG. 4, and the determination unit 620 determines the type of the odor source as pet excrement, the control unit 640 controls the air conditioning apparatus 100 such that the air conditioning apparatus 100 performs the cooling operation. The control unit 640 also controls the air conditioning apparatus 100 (particularly, the air direction adjustment flap 17) so as to avoid wind generated by the indoor unit 10 from directly hitting on the odor source. In the case where the indoor unit of the air conditioning apparatus 100 is the indoor unit 10' (see FIG. 5) capable of performing the air curtain operation, the control unit 640 controls the air conditioning apparatus 100 (particularly the air direction adjustment flap on the blow-out port 13 in the indoor unit 10') such that the air conditioning apparatus 100 performs the air curtain operation.

After the commencement of the first control on the air conditioning apparatus 100, when the determination unit 620 determines that the first odor source becomes absent from the target space R, based on at least one of an image or a sound of the target space R acquired by the acquisition unit 610 (step S4), the processing of the odor control on the target space R proceeds to step S5.

In step S5, the control unit 640 refers to the pattern database 672 of the storage unit 670 to determine whether second control is prepared for the type of the odor source (i.e., the first odor source) determined by the determination unit 620 in step S1. When second control is prepared for the first odor source, the processing of the odor control on the target space R proceeds to step S6.

On the other hand, when the control unit 640 determines in step S5 that second control is not prepared for the first odor source, the odor control on the target space R ends. When the determination unit 620 determines again that an odor source of any type is present in the target space R, the processing in and subsequent to step S2 is carried out again.

In step S6, the control unit 640 commences the second control on the air conditioning apparatus 100. In other words, the control unit 640 controls the air conditioning apparatus 100, based on the content of the second control prepared for the first odor source. The control unit 640 implements the second control on the air conditioning apparatus 100 for a predetermined time (e.g., 10 minutes), and then terminates the second control. The processing of the odor control on the target space R thus ends. When the determination unit 620 determines again that an odor source of any type is present in the target space R, the processing in and subsequent to step S2 is carried out again.

### (4) Features

### (4-1)

The spatial environment management system 1 according to the first embodiment includes the acquisition unit 610, the determination unit 620, the air conditioning apparatus 100 as an example of an device (an odor treatment device), and the control unit 640. The acquisition unit 610 acquires at least one of an image of the target space R and a sound of the target space R. In the first embodiment, the acquisition unit 610 acquires both an image of the target space R and a sound of the target space R. The determination unit 620 determines whether an odor emitting object (an odor source) is present in the target space R, based on at least one of the image or the sound acquired by the acquisition unit 610. The air conditioning apparatus 100 includes at least the indoor fan 12 as an airflow producing mechanism. The control unit 640 controls the air conditioning apparatus 100 such that the air conditioning apparatus 100 treats an odor when the determination unit 620 determines that an odor source is present in the target space R.

Since presence of an odor emitting object is determined from an image and a sound, the control unit 640 promptly controls the air conditioning apparatus 100 including the airflow producing mechanism and the deodorizing mechanism even when an odor sensor is distant from the odor emitting object.

Even when there is no odor sensor in the target space R (e.g., even when the spatial environment management system 1 includes no odor sensor), the control unit 640 appropriately operates the air conditioning apparatus 100 on an odor.

### (4-2)

In the spatial environment management system 1 according to the first embodiment, the determination unit 620 determines a type of an odor source based on at least one of an image and a sound acquired by the acquisition unit 610. When the determination unit 620 determines that an odor source is present in the target space R, the control unit 640 controls the air conditioning apparatus 100 in accordance with a content set based on the type of the odor source determined by the determination unit 620.

According to this configuration, the control unit 640 appropriately operates the air conditioning apparatus 100 in consideration of a type of an odor source.

### (4-3)

In the spatial environment management system 1 according to the first embodiment, the control unit 640 has the plurality of control patterns for the air conditioning apparatus 100. The spatial environment management system 1 includes the storage unit 670 storing types of odor sources and the control patterns corresponding to the types with the types of odor sources and the control patterns associated with each other. The control unit 640 controls the air conditioning apparatus 100 in accordance with the control pattern corresponding to the type of the odor source determined by the determination unit 620, based on the information stored in the storage unit 670.

According to this configuration, the storage unit 670 stores the types of the odor sources and the control patterns for the air conditioning apparatus 100 with the types of the odor sources and the control patterns associated with each other. The control unit 640 therefore promptly and appropriately operates the air conditioning apparatus 100 in accordance with the type of the odor source.

### (4-4)

The spatial environment management system 1 according to the first embodiment includes the identification unit 630. The identification unit 630 identifies, when the determination unit 620 determines that an odor source is present in the target space R, a position of the odor source, based on at least one of an image and a sound acquired by the acquisition unit 610. The control unit 640 controls the air conditioning apparatus 100 in accordance with a content set based on the position of the odor source identified by the identification unit 630.

According to this configuration, since the control unit 640 controls the air conditioning apparatus 100 based on a position of an odor source, the control unit 640 appropriately operates the air conditioning apparatus 100 on the odor.

### (4-5)

In the spatial environment management system 1 according to the first embodiment, the control unit 640 controls at least one of the ON/OFF state of the air conditioning apparatus 100, a direction of air blown out from the air conditioning apparatus 100 (specifically, the indoor unit 10) and a volume of the air blown out from the air conditioning apparatus 100 (specifically, the indoor unit 10).

According to this configuration, the control unit 640 appropriately operates the air conditioning apparatus 100 on the odor by changing the ON or OFF state of the air conditioning apparatus 100, the direction of air from the air conditioning apparatus 100, and the volume of air from the air conditioning apparatus 100.

### (4-6)

In the spatial environment management system 1 according to the first embodiment, when the determination unit 620 determines that an odor source is present in the target space R, the control unit 640 controls the air conditioning apparatus 100 in accordance with a content set based on the action of a person on the odor source.

According to this configuration, since the control unit 640 controls the air conditioning apparatus 100 based on the action of a person on an odor source, the control unit 640 appropriately operates the air conditioning apparatus 100 on the odor.

### (4-7)

In the spatial environment management system 1 according to the first embodiment, when the determination unit 620 determines that an odor source (e.g., a first odor source) is present in the target space R, the control unit 640 commences first control on the air conditioning apparatus 100 such that the air conditioning apparatus 100 treats the odor. When the determination unit 620 determines that the first odor source is absent from the target space R after the commencement of the first control, the control unit 640 implements second control on the air conditioning apparatus 100 such that the air conditioning apparatus 100 treats the odor. The second control is different from the first control.

According to this configuration, the air conditioning apparatus 100 further performs a predetermined operation even after the odor source becomes absent from the target space R. This configuration thus suppresses occurrence of a state in which the odor still remains in the target space R although the odor source becomes absent from the target space R.

### (4-8)

In the spatial environment management system 1 according to the first embodiment, the device (the odor treatment device) includes the air conditioning apparatus 100. When the determination unit 620 determines that an odor source is present in the target space R, the control unit 640 controls the air conditioning apparatus 100 such that the air conditioning apparatus 100 performs one of the cooling operation, the dehumidifying operation, and the heating operation to treat the odor.

According to this configuration, since the cooling operation, the dehumidifying operation or the heating operation is utilized for treating an odor, the odor is removed from the target space R with ease.

In addition, a reduction in an odor which an odor source emits is also expected by the utilization of the cooling operation, dehumidifying operation or heating operation for treating the odor. For example, in a case where an odor source is a person who is sweating, the air conditioning apparatus 100 performs the cooling operation to suppress the sweat. In a case where an odor source is wet laundry hung indoors, the air conditioning apparatus 100 performs the heating operation or the dehumidifying operation to promote drying of the laundry, thereby inhibiting the growth of bacteria and suppressing emission of an odor. In a case where an odor source is mold on, for example, a wall of a room, the air conditioning apparatus 100 performs the dehumidifying operation or the heating operation to inhibit the growth of mold and to suppress emission of an odor.

### (5) Modifications

Next, a description will be given of modifications of the first embodiment. It should be noted that some of or all of the configurations in the respective modifications may be combined with some of or all of the configurations in the other modifications insofar as there are no consistencies.

### (5-1) Modification 1A

In the first embodiment, the control apparatus 600 of the spatial environment management system 1 includes the controller 50 of the air conditioning apparatus 100 and the server 200. However, the control apparatus 600 is not limited to this configuration. For example, the spatial environment management system 1 does not include the server 200, and only the controller 50 may function as the control apparatus 600. Alternatively, the server 200 may mainly function as the control apparatus 600 and control the air conditioning apparatus 100 by transmitting an instruction to the controller 50.

### (5-2) Modification 1 B

In the first embodiment, the air conditioning apparatus 100 as the odor treatment device has the plurality of control patterns. However, the air conditioning apparatus 100 is not limited to this configuration. For example, when the determination unit 620 determines that an odor source is present in the target space R, the control unit 640 may implement the same control irrespective of a type of the odor source (e.g., may avoid wind from hitting on the odor source). In a case where the odor treatment device is a ventilating fan, when the determination unit 620 determines that an odor source is present in the target space R, the control unit 640 may implement the same control irrespective of a type of the odor source (e.g., may turn on the ventilating fan). In the case where the control unit 640 does not change the control on the odor treatment device depending on a type of an odor source, the determination unit 620 does not necessarily determine a type of an odor source.

### (5-3) Modification 1C

In the first embodiment, the determination unit 620 determines whether an odor source is present in the target space R, based on both an image of the target space R and a sound of the target space R. However, the determination unit 620 is not limited to this configuration. The determination unit 620 may determine whether an odor source is present in the target space R, based on either an image or a sound of the target space R, for example, based on only an image of the target space R. For example, in a case where a sound of the target space R is not used for a determination by the determination unit 620, the spatial environment management system 1 does not necessarily include the microphone 420 and the sound acquisition unit 614.

### (5-4) Modification 1 D

In the first embodiment, the spatial environment management system 1 includes the camera 410 and the microphone 420. However, the spatial environment management system 1 is not limited to this configuration. The spatial environment management system 1 may acquire an image captured by a camera provided separately from the system (e.g., a mobile information terminal of the user) and a sound collected by a microphone provided separately from the system (e.g., the mobile information terminal of the user).

### (5-5) Modification 1 E

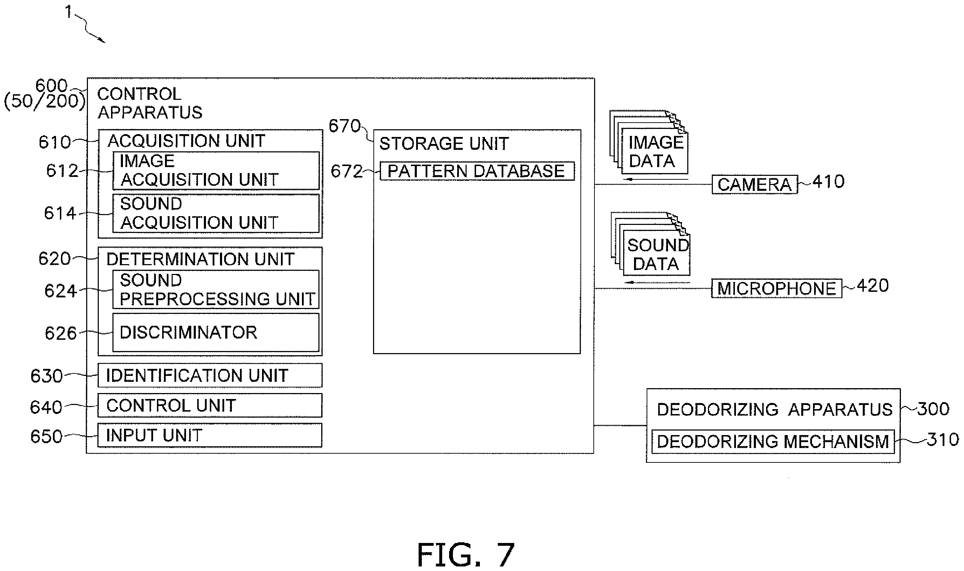

In the first embodiment, the spatial environment management system 1 includes one odor treatment device. Alternatively, the spatial environment management system 1 may include a plurality of odor treatment devices. As illustrated in FIG. 7, for example, the spatial environment management system 1 may further include a deodorizing apparatus 300 including a deodorizing mechanism 310, in addition to the air conditioning apparatus 100.

In a case where multiple odor treatment devices are present in the target space R, information indicating which of the odor treatment devices (e.g., the air conditioning apparatus 100 and the deodorizing apparatus 300) is operated may be contained as a content of a control pattern for an odor treatment device by the control unit 640. For example, the control unit 640 may cause the air conditioning apparatus 100 to perform a predetermined operation when a type of an odor source is X, and may cause the deodorizing apparatus 300 to perform a predetermined operation when a type of an odor source is Y. With this configuration, the spatial environment management system 1 appropriately operates the odor treatment device in accordance with a type of an odor source.

In the case where the multiple odor treatment devices are present in the target space R, the control unit 640 may control the odor treatment devices such that a corresponding one of the odor treatment devices operates in accordance with a position of an odor source identified by the identification unit 630. For example, the control unit 640 may control the multiple odor treatment devices (e.g., the air conditioning apparatus 100 and the deodorizing apparatus 300) such that a closer one of the odor treatment devices to the odor source operates.

### (5-6) Modification 1F

In the first embodiment, the determination unit 620 determines whether an odor source is present in the target space R and a type of the odor source, using the discriminator 626 learned based on machine learning. However, the determination unit 620 does not necessarily make the determination using the discriminator 626. For example, when a rule (e.g., a rule that when an image or a sound has a feature α, an odor source of a type β is present) is manually given to the control apparatus 600 in advance, the determination unit 620 may determine whether an odor source is present in the target space R and a type of the odor source, based on this rule.

### <Second Embodiment

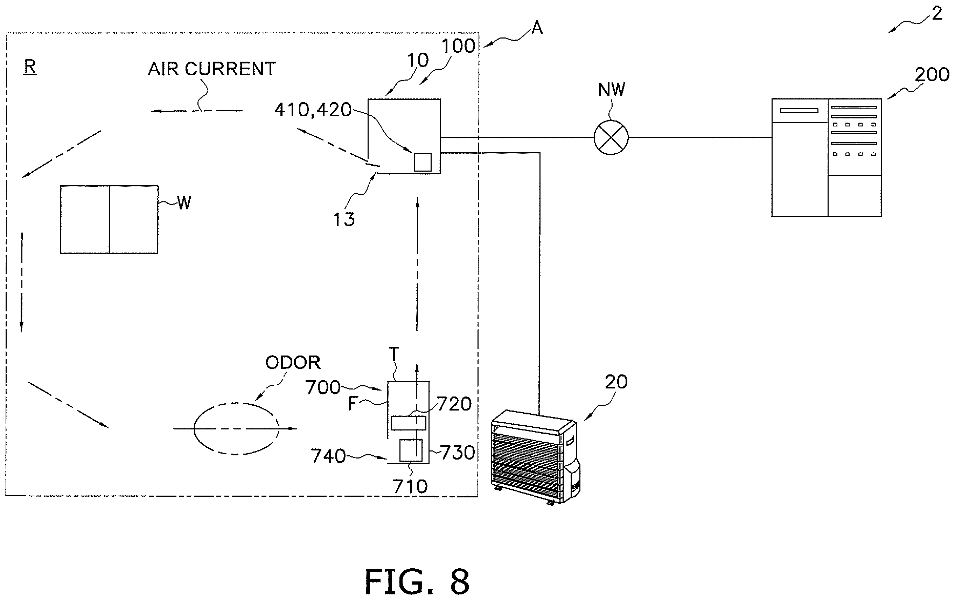

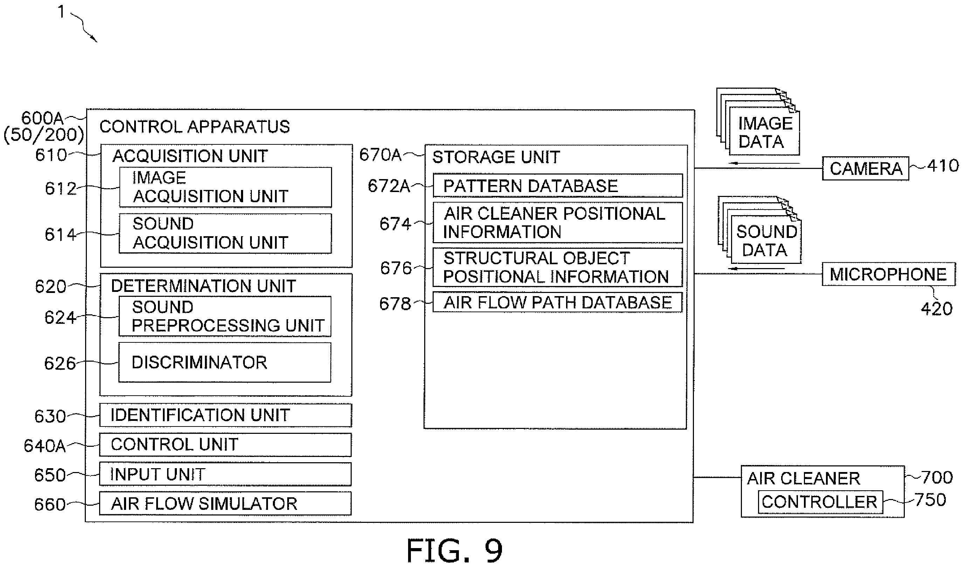

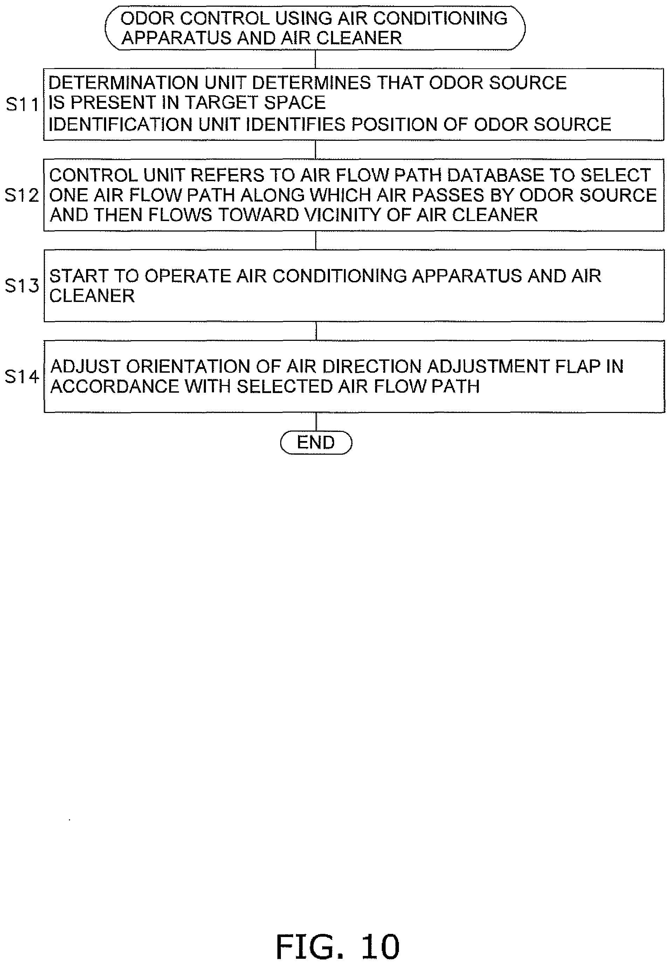

With reference to FIGS. 8 to 10, a description will be given of a spatial environment management system 2 according to a second embodiment. FIG. 8 is a schematic configuration diagram of the spatial environment management system 2. FIG. 9 is a schematic block diagram of a control apparatus 600A of the spatial environment management system 2. FIG. 10 is an exemplary flowchart of odor control on a target space R by the spatial environment management system 2 using a cooperation control pattern to be described later.

The configuration of the spatial environment management system 1 according to the first embodiment as well as the configurations of Modifications 1A to 1 F may be combined with the configuration of the spatial environment management system 2 according to the second embodiment insofar as there are no inconsistencies. On the contrary, the following configuration of the spatial environment management system 2 may be combined with the spatial environment management system 1 according to the first embodiment as well as the configurations of Modifications 1A to 1F insofar as there are no inconsistencies.

The first embodiment mainly describes, as an example, the spatial environment management system in the case where a single odor treatment device treats an odor in the target space R and in the case where multiple odor treatment devices treat an odor in the target space R independently of one another. In contrast to this, the spatial environment management system 2 according to the second embodiment is mainly different from the spatial environment management system 1 according to the first embodiment in that multiple odor treatment devices treat an odor in the target space R in cooperation with one another, in addition to the aspects of the first embodiment or in place of the aspects of the first embodiment.

The spatial environment management system 2 mainly includes: an air conditioning apparatus 100 and an air cleaner 700 as examples of odor treatment devices to be installed in the target space R; and a server 200 connected to a controller 50 of the air conditioning apparatus 100 via a network NW so as to establish communications with the controller 50 (see FIG. 8). The air cleaner 700 is an example of an odor reducer configured to reduce an odor. In the spatial environment management system 2, two odor treatment devices (e.g., the air conditioning apparatus 100 and the air cleaner 700) treat an odor in the target space R in cooperation with each other. However, the number of cooperative odor treatment devices is not limited to two. For example, three or more odor treatment devices may treat an odor in the target space R in cooperation with one another.

Of the two odor treatment devices, the air conditioning apparatus 100 has already been described in the first embodiment; therefore, the description thereof will not be given here.

The air cleaner 700 is an apparatus configured to remove dust in the air. The air cleaner 700 is also an apparatus configured to remove an odor substance in the air. In other words, the air cleaner 700 is an apparatus configured to reduce an odor. A description on a dust removing function of the air cleaner 700 will not be given here. The air cleaner 700 mainly includes a fan 710, a deodorizing filter 720, a casing 730, and a controller 750. The fan 710 is an example of an airflow producing mechanism. The deodorizing filter 720 is an example of a deodorizing mechanism. The casing 730 houses therein the fan 710 and the deodorizing filter 720. The controller 750 includes an MCU mainly including a CPU, a memory, an input-output port, and the like (not illustrated). The controller 750 is a control apparatus configured to control the operations of the various components in the air cleaner 700. The controller 750 controls, for example, an ON or OFF state of the fan 710 and a volume of air from the fan 710.

When the controller 750 operates the fan 710, the air cleaner 700 sucks air into the casing 730 through a suction port 740 formed in an outer surface of the casing 730. The air taken into the casing 730 passes the deodorizing filter 720, and then is blown out of the casing 730 through a blow-out port (not illustrated) formed in the outer surface of the casing 730. An odor component in the air is adsorbed on and removed by the deodorizing filter 720 when passing the deodorizing filter 720. In the second embodiment, for example, the casing 730 has in its front surface F the suction port 740, and also has in its top surface T the blow-out port (not illustrated) (see FIG. 8).

The air cleaner 700 may further include an odor sensor (not illustrated) in addition to the foregoing configurations. The controller 750 may operate the fan 710 when the odor sensor detects an odor. In addition, the controller 750 may adjust the volume of air from the fan 710 in accordance with a degree of an odor detected by the odor sensor.

The typical structure and function of the air cleaner 700 are publicly known; therefore, the additional description of the air cleaner 700 will not be given here.

In a manner similar to that described in the first embodiment, the controller 50 of the air conditioning apparatus 100 and the server 200 constitute a control apparatus 600A of the spatial environment management system 2. The control apparatus 600A is different in part of processes to be executed from the control apparatus 600 in the first embodiment. However, the control apparatus 600A and the control apparatus 600 have many common features in their physical configurations and functions. In the following, therefore, a description will be mainly given of a difference between the control apparatus 600A in the second embodiment and the control apparatus 600 in the first embodiment, and a description on the common features between the control apparatus 600A and the control apparatus 600 will not be basically given here.

Preferably, the control apparatus 600A is connected to the controller 750 of the air cleaner 700 via a wired communication line or a wireless network so as to establish communications with the controller 750. Preferably, the control apparatus 600A is capable of transmitting, to the controller 750 of the air cleaner 700, at least an instruction to operate the air cleaner 700 and an instruction to stop the air cleaner 700.

As described above, the controller 50 of the air conditioning apparatus 100 and the server 200 constitute the control apparatus 600A. According to another aspect, the controller 750 of the air cleaner 700, the controller 50 of the air conditioning apparatus 100, and the server 200 may constitute the control apparatus 600A.

### (1-1) Control Apparatus

A description will be given of functions of the control apparatus 600A.

The control apparatus 600A mainly includes as its functional unit an acquisition unit 610, a determination unit 620, an identification unit 630, a control unit 640A, an input unit 650, an air flow simulator 660, and a storage unit 670A.

The functions of the acquisition unit 610, determination unit 620, identification unit 630, and input unit 650 are similar to the functions of the acquisition unit 610, determination unit 620, identification unit 630, and input unit 650 of the control apparatus 600 in the first embodiment; therefore, the description thereof will not be given here.

### (1-1-1) Storage Unit

A description will be given of the storage unit 670A of the control apparatus 600A.

The storage unit 670A of the control apparatus 600A is different from the storage unit 670 of the control apparatus 600 in the first embodiment in the following respects.

The storage unit 670A has a pattern database 672A storing a cooperation control pattern as a control content corresponding to a predetermined odor source, in place of or in addition to the information stored in the pattern database 672 of the storage unit 670 described in the first embodiment. The cooperation control pattern used herein means a control pattern to be used when the control unit 640A causes the air conditioning apparatus 100 and the air cleaner 700 to treat an odor in the target space R in cooperation with each other as will be described later.

The storage unit 670A also stores air cleaner positional information 674. The air cleaner positional information 674 is positional information on the air cleaner 700 located in the target space R. For example, the air cleaner positional information 674 is relative positional information on the air cleaner 700 relative to the air conditioning apparatus 100. In other words, the air cleaner positional information 674 is information indicating a direction of the air cleaner 700 relative to the air conditioning apparatus 100 and a distance from the air cleaner 700 to the air conditioning apparatus 100.

It should be noted however that the air cleaner positional information 674 is not necessarily relative positional information on the air cleaner 700. For example, the air cleaner positional information 674 may be information on an absolute position of the air cleaner 700 in the target space R. In this case, preferably, the storage unit 670A stores information on an absolute position of the air conditioning apparatus 100 in the target space R so as to grasp the relative positional relationship between the air conditioning apparatus 100 and the air cleaner 700.

The air cleaner positional information 674 is, for example, information which a user or a worker who installs the spatial environment management system 2 inputs through the input unit 650. In this case, the input unit 650 functions as a position acquisition unit configured to acquire a position of the air cleaner 700 as an example of the odor reducer.

According to another aspect, the identification unit 630 may function as a position acquisition unit to acquire the air cleaner positional information 674 based on at least one of an image or a sound of the target space R acquired by the acquisition unit 610 as will be described later.

For example, the determination unit 620 determines whether the air cleaner 700 is present in the target space R based on an image of the target space R captured by the camera 410 and acquired by the acquisition unit 610. A determination as to whether the air cleaner 700 is present in the target space R is made using a method similar to the method for a determination as to whether an odor source is present in the target space R. When the determination unit 620 determines that the air cleaner 700 is present in the target space R, the identification unit 630 identifies a position of the air cleaner 700 in an actual space, using a calculation formula for associating a position of a region where the air cleaner 700 comes out in an image of the target space R with a position of the air cleaner 700 in an actual space, a database storing the correlation, and others.

The determination unit 620 may also determine whether the air cleaner 700 is present in the target space R, based on a sound of the target space R collected by the microphone 420 and transmitted to the acquisition unit 610. For example, the determination unit 620 determines whether the air cleaner 700 is present in the target space R, based on whether a sound of the target space R contains an operating sound of the air cleaner 700. When it is determined that the air cleaner 700 is present in the target space R, the identification unit 630 may ascertain a direction of the operating sound of the air cleaner 700 and identify the direction as a position of the air cleaner 700.

The storage unit 670A also stores structural object positional information 676. The structural object positional information 676 is positional information on a position of a wall of the target space R and/or a position of an obstacle that hinders a flow of air blown out from the air conditioning apparatus 100. Examples of the obstacle that hinders a flow of air blown out from the air conditioning apparatus 100 may include, but not limited to, large furniture and home electrical equipment. For example, the structural object positional information 676 is relative positional information on the wall and/or the obstacle relative to the air conditioning apparatus 100.

The structural object positional information 676, which is similar to the air cleaner positional information 674, may be information acquired by the input unit 650 or may be information acquired by the identification unit 630 based on, for example, an image captured by the camera 410.

The storage unit 670A includes an air flow path database 678 to be described later.

### (1-1-2) Air Flow Simulator

Next, a description will be given of the air flow simulator 660 of the control apparatus 600A.

The air flow simulator 660 is a functional unit configured to, in a case where the air direction adjustment flap 17 of the air conditioning apparatus 100 is controlled such that the air conditioning apparatus 100 blows out air in a certain direction, simulate a route of the air flowing through the target space R.

The air flow simulator 660 simulates a route of the air flowing through the target space R in a case when the air conditioning apparatus 100 blows out air in a certain direction, based on the structural object positional information 676. Preferably, a result of simulation by the air flow simulator 660 (i.e., an air flow path) is stored in the air flow path database 678 of the storage unit 670A, depending on a direction of air from the air conditioning apparatus 100. In other words, preferably, an air flow path obtained from the simulation by the air flow simulator 660 is stored in the air flow path database 678 of the storage unit 670A with the air flow path associated with an orientation of the air direction adjustment flap 17.

### (1-1-3) Control Unit

Next, a description will be given of the control unit 640A of the control apparatus 600A.

In addition to or in place of the control by the control unit 640 described in the first embodiment, the control unit 640A of the control apparatus 600A is capable of causing the air conditioning apparatus 100 and the air cleaner 700 to treat an odor in the target space R in cooperation with each other. In other words, the pattern database 672A of the storage unit 670A stores, as a control content corresponding to a certain odor source, a control pattern (a cooperation control pattern) which the control unit 640A causes the air conditioning apparatus 100 and the air cleaner 700 to treat an odor in the target space R in cooperation with each other.

In accordance with the cooperation control pattern, the control unit 640A controls the operations of the air conditioning apparatus 100 and air cleaner 700 as illustrated in, for example, a flowchart of FIG. 10.

It is assumed that the determination unit 620 determines that an odor source for which the cooperation control pattern is used (referred to as a second odor source) is present in the target space R, and the identification unit 630 identifies a position of the second odor source in the target space R (step S11).

When the condition in step S11 is established, the control unit 640A refers to the air flow path database 678. The control unit 640A selects one air flow path along which air passes through or by a position of the second odor source, and then flows toward the air cleaner 700, from among the air flow paths stored in the air flow path database 678 (step S12). An air flow path along which air flows toward the air cleaner 700 used herein includes an air flow path along which air flows toward the vicinity of the air cleaner 700. For example, the control unit 640A selects an air flow path along which air passes through the position of the second odor source or passes by the nearest position of the second odor source, from among air flow paths along which air flows toward the air cleaner 700, the air flow paths being stored in the air flow path database 678. For example, the control unit 640A also selects an air flow path along which air directly flows toward the air cleaner 700 or passes by the nearest position of the air cleaner 700, from among air flow paths along which air passes through the position of the second odor source, the air flow paths being stored in the air flow path database 678.

In step S12, preferably, the control unit 640A selects an air flow path in consideration of a position of the suction port 740 in the casing 730 of the air cleaner 700. Specifically, the control unit 640A preferably selects one air flow path along which air passes through or by the position of the second odor source, and then flows toward the air suction port 740 in the casing 730 of the air cleaner 700, from among the air flow paths stored in the air flow path database 678. An air flow path along which air flows toward the air suction port 740 in the air cleaner 700 used herein includes an air flow path along which air flows toward the vicinity of the suction port 740 in the air cleaner 700. In the second embodiment, for example, the casing 730 of the air cleaner 700 has in its front surface F the suction port 740. Therefore, the control unit 640A selects one air flow path along which air passes through or by the position of the second odor source, and then flows toward the front surface F of the casing 730 of the air cleaner 700, from among the air flow paths stored in the air flow path database 678. In the case where the control unit 640A selects an air flow path in consideration of the position of the suction port 740 in the casing 730, preferably, the air cleaner positional information 674 stored in the storage unit 670A contains positional information of the suction port 740 acquired by the input unit 650 or the identification unit 630.

Next, the control unit 640A starts to operate the air conditioning apparatus 100 and the air cleaner 700 (step S13).

Next, the control unit 640A refers to the air flow path database 678, reads an orientation of the air direction adjustment flap 17 corresponding to the air flow path selected in step S12, and adjusts the orientation of the air direction adjustment flap 17 of the air conditioning apparatus 100 (step S14). The control unit 640A may adjust a volume of air from the air conditioning apparatus 100 in consideration of, for example, a distance between the air conditioning apparatus 100 and the air cleaner 700. For example, the control unit 640A increases the volume of air from the air conditioning apparatus 100 as the air conditioning apparatus 100 is farther from the air cleaner 700.

The cooperation control pattern is used in cooking generating a strong odor and smoke, such as Korean barbeque. In other words, the cooperation control pattern is used, for example, when the determination unit 620 determines that a second odor source is present, based on the presence of smoke. The cooperation control pattern may also be used, for example, when the determination unit 620 determines that a second odor source is present, based on, for example, the presence of cookware for Korean barbeque.

The air conditioning apparatus 100 guides an odor or smoke to the air cleaner 700 in accordance with the cooperation control pattern. This configuration thus suppresses occurrence of a situation in which the indoor unit 10 of the air conditioning apparatus 100 directly sucks an odor or smoke through the suction port and, as a result, also suppresses occurrence of a situation in which the indoor unit 10 spreads the odor or smoke throughout the target space R. In addition, since this configuration suppresses the occurrence of the situation in which the indoor unit 10 directly sucks an odor or smoke through the suction port, this configuration also suppresses occurrence of a situation in which the filter or the like of the indoor unit 10 adsorbs the odor so that the odor remains in the target space R for a long period of time.

The cooperation control pattern is also effective, for example, in a case where the air conditioning apparatus 100 is at a standstill for a long period of time and in a case where the air conditioning apparatus 100 is operated without being dried after dew or frost adheres to an indoor heat exchanger (not illustrated) of the indoor unit 10 due to the cooling operation of the air conditioning apparatus 100. In the foregoing cases, for example, the control unit 640A refers to the air flow path database 678. The control unit 640A selects one air flow path along which air flows toward the air cleaner 700, from among the air flow paths stored in the air flow path database 678. Next, the control unit 640A starts to operate the air conditioning apparatus 100 and the air cleaner 700. At this time, the control unit 640A refers to the air flow path database 678, reads an orientation of the air direction adjustment flap 17 corresponding to the selected air flow path, and adjusts the orientation of the air direction adjustment flap 17 of the air conditioning apparatus 100. The control by the control unit 640A suppresses the spread of an odor throughout the target space R due to odor generated at the air conditioning apparatus 100.

### (2) Features

The spatial environment management system 2 according to the second embodiment has the following features.

### (2-1)

The spatial environment management system 2 includes the position acquisition unit configured to acquire information on a position of the air cleaner 700. The air cleaner 700 is an example of an odor reducer configured to reduce an odor.

The position acquisition unit is, for example, the input unit 650 configured to receive an input of information. For example, the identification unit 630 may function as the position acquisition unit to acquire the information on the position of the air cleaner 700 based on at least one of an image or a sound of the target space R acquired by the acquisition unit 610.

The control unit 640A controls the air conditioning apparatus 100 such that air passes through or by a position of a second odor source, and then flows toward the air cleaner 700. The air conditioning apparatus 100 is an example of an odor treatment device including the indoor fan 12 as an airflow producing mechanism.

In the spatial environment management system 2 according to the second embodiment, an odor in the target space R is efficiently reduced by a combination of the air conditioning apparatus 100 as an example of the device with the air cleaner 700 as an example of the odor reducer.

In the case where the identification unit 630 functions as the position acquisition unit to acquire the information on the position of the air cleaner 700 based on the image of the target space R acquired by the acquisition unit 610, the user or the like does not necessarily grasp the position of the air cleaner 700 and input the grasped information through the input unit 650. The configuration in which the identification unit 630 acquires the information on the position of the air cleaner 700 based on the image of the target space R acquired by the acquisition unit 610 is adaptive even when the position of the air cleaner 700 is changed.

### (2-2)

In the spatial environment management system 2, preferably, the position acquisition unit configured to acquire information on a position of the air cleaner 700 further acquires information on a position of the air suction port 740 in the air cleaner 700. For example, preferably, the identification unit 630 functions as the position acquisition unit to acquire information on a position of the air suction port 740 in the air cleaner 700 based on an image of the target space R acquired by the acquisition unit 610. The control unit 640A controls the air conditioning apparatus 100 such that air passes through or by a position of a second odor source, and then flows toward the suction port 740 in the air cleaner 700.

According to this configuration, an odor substance is taken into the air cleaner 700 with ease as compared with a case where no consideration is given to the position of the suction port 740. An odor in the target space R is thus reduced with ease.

### (3) Modifications

Next, a description will be given of modifications of the second embodiment. It should be noted that some of or all of the configurations in the respective modifications may be combined with some of or all of the configurations in the other modifications insofar as there are no consistencies. The following modifications may be combined with the configuration of the spatial environment management system 1 according to the first embodiment as well as the configurations of Modifications 1A to 1F insofar as there are no inconsistencies.

### (3-1) Modification 2A

In the second embodiment, the control apparatus 600A and the controller 750 of the air cleaner 700 are connected so as to establish communications with each other, and the control apparatus 600A transmits to the controller 750 an instruction to operate the air cleaner 700.

According to another aspect, the control apparatus 600Adoes not necessarily establish communications with the controller 750. For example, the air cleaner 700 is an apparatus configured to monitor an air environment using an odor sensor and to automatically operate based on a result of detection by the odor sensor. In other words, the air cleaner 700 is a device that is provided independently of the spatial environment management system 2 and is configured to autonomously operate. In this case, when an airflow produced by the air conditioning apparatus 100 feeds an odor substance to the vicinity of the air cleaner 700, the air cleaner 700 detects the odor to start its autonomous operation.

### (3-2) Modification 2B

Even in the presence of an odor source (i.e., a second odor source) for which the cooperation control pattern should be used in view of the information stored in the pattern database 672A, the control unit 640A does not necessarily operate at least one of the air conditioning apparatus 100 and the air cleaner 700 when a predetermined condition is established.

For example, the control unit 640A does not necessarily operate the air cleaner 700 when a window W of the target space R is open even in the presence of a second odor source. It should be noted that whether the window W of the target space R is open may be detected, for example, from an image captured by the camera 410 or using a sensor attached to the window.

### (3-3) Modification 2C

In the second embodiment, the air cleaner 700 is an odor reducer. Examples of the odor reducer may include, but not limited to, a deodorizing apparatus, and a ventilator configured to discharge air from the target space R.

### (3-4) Modification 2D

In the second embodiment, the air cleaner 700 as an odor reducer treats an odor in cooperation with the air conditioning apparatus 100. However, the spatial environment management system 2 is not necessarily a system in which multiple devices treat an odor in cooperation with one another.

Specifically, the odor reducer is not necessarily a device. For example, the odor reducer may be an exhaust port formed in the target space R to communicate the target space R with the outside. The odor reducer may also be an open window W of the target space R. The control unit 640A may control the air conditioning apparatus 100 such that air passes through or by a position of a second odor source, and then flows toward the open window W as the odor reducer.

Information on a position of the window W may be acquired by the input unit 650 or may be acquired by the identification unit 630 as described above. In addition, whether the window W of the target space R is open may be detected, for example, from an image captured by the camera 410 or using a sensor attached to the window.

The foregoing description concerns embodiments and modifications of the disclosure. It will be understood that numerous modifications and variations may be made without departing from the gist and scope of the disclosure in the appended claims.

### INDUSTRIAL APPLICABILITY

The present disclosure is widely applicable to and useful for a spatial environment management system including a device that includes at least one of an airflow producing mechanism and a deodorizing mechanism for treating an odor.

### REFERENCE SIGNS LIST

1, 2: spatial environment management system
12: indoor fan (airflow producing mechanism)
100: air conditioning apparatus (device)
300: deodorizing apparatus (device)
310: deodorizing mechanism
610: acquisition unit
620: determination unit
630: identification unit (position acquisition unit)
640, 640A: control unit
650: input unit (position acquisition unit)
670, 670A: storage unit
700: air cleaner (device, odor reducer)
710: fan (airflow producing mechanism)
720: deodorizing filter (deodorizing mechanism)
740: suction port
W: window (odor reducer)
R: target space

### CITATION LIST

### PATENT LITERATURE

Patent Literature 1: JP 10-170422 A

## Claims

1. A spatial environment management system (1, 2) comprising:
an acquisition unit (610) configured to acquire at least one of an image of a target space (R) and a sound of the target space;
a determination unit (620) configured to determine whether an odor emitting object is present in the target space, based on at least one of the image and the sound acquired by the acquisition unit;
a device (100, 300, 700) including at least one of an airflow producing mechanism (12) and a deodorizing mechanism (310); and
a control unit (640, 640A) configured to control the device such that the device treats an odor when the determination unit determines that the odor emitting object is present in the target space.

2. The spatial environment management system according to claim 1, wherein
the determination unit is further configured to determine a type of the odor emitting object, based on at least one of the image and the sound acquired by the acquisition unit, and
when the determination unit determines that the odor emitting object is present in the target space, the control unit is configured to control the device in accordance with a content set based on the type of the odor emitting object determined by the determination unit.

3. The spatial environment management system according to claim 2, wherein
the control unit has a plurality of control patterns for the device,
the spatial environment management system further comprising:
a storage unit (670, 670A) configured to store types of odor emitting objects and the control patterns corresponding to the types of odor emitting objects with the types and the control patterns being associated with each other,
wherein
the control unit is configured to control the device in accordance with the control pattern corresponding to the type of the odor emitting object, determined by the determination unit, based on the information stored in the storage unit.

4. The spatial environment management system according to any one of claims 1 to 3, further comprising:
an identification unit (630) configured to identify, when the determination unit determines that the odor emitting object is present in the target space, a position of the odor emitting object, based on at least one of the image and the sound acquired by the acquisition unit,
wherein
the control unit is configured to control the device in accordance with a content set based on the position of the odor emitting object identified by the identification unit.

5. The spatial environment management system (2) according to claim 4, further comprising:
a position acquisition unit (630, 650) configured to acquire information on a position of an odor reducer (700, W) configured to reduce an odor,
wherein
the device includes at least the airflow producing mechanism, and
the control unit (640A) is configured to control the device such that air passes through or by the position of the odor emitting object, and then flows toward the odor reducer.

6. The spatial environment management system according to claim 5, wherein
the position acquisition unit (630) is configured to acquire the information on the position of the odor reducer based on the image acquired by the acquisition unit.

7. The spatial environment management system according to claim 6, wherein
the odor reducer includes an air cleaner (700) including a deodorizing mechanism (720) and an airflow producing mechanism (710),
the position acquisition unit is further configured to acquire information on a position of an air suction port (740) formed in the air cleaner, based on the image acquired by the acquisition unit, and
the control unit is configured to control the device such that air passes through or by the position of the odor emitting object, and then flows toward the suction port in the air cleaner.

8. The spatial environment management system according to claim 7, wherein
when the determination unit determines that the odor emitting object is present in the target space, the control unit is further configured to control the air cleaner such that the air cleaner treats the odor.

9. The spatial environment management system according to any one of claims 1 to 8, wherein
the control unit is configured to control at least one of an ON/OFF state of the device, a direction of air blown out from the device and a volume of air blown out from the device.

10. The spatial environment management system according to any one of claims 1 to 9, wherein
the device includes at least one of an air conditioning apparatus, an air cleaner, an electric fan, a ventilator and a deodorizing apparatus.

11. The spatial environment management system according to any one of claims 1 to 10, wherein
the odor emitting object as a target of determination by the determination unit includes at least one of food, life and excrement.

12. The spatial environment management system according to any one of claims 1 to 11, wherein
when the determination unit determines that the odor emitting object is present in the target space, the control unit is configured to control the device in accordance with a content set based on action of a person on the odor emitting object.

13. The spatial environment management system according to any one of claims 1 to 12, wherein
when the determination unit determines that a first object having an odor is present in the target space, the control unit is configured to commence first control on the device such that the device treats the odor, and
when the determination unit determines that the first object becomes absent from the target space after the commencement of the first control, the control unit is configured to implement second control different from the first control on the device such that the device treats the odor.

14. The spatial environment management system according to claim 1, wherein
the device (100) includes an air conditioning apparatus, and
when the determination unit determines that the odor emitting object is present in the target space, the control unit is configured to control the air conditioning apparatus such that the air conditioning apparatus performs one of a cooling operation, a dehumidifying operation, and a heating operation to treat the odor.
